(19) Europäisches Patentamt
European Patent Office
Office européen des brevets

(11) Publication number: 0 106 988 A1

# (12) EUROPEAN PATENT APPLICATION

(21) Application number: 83108640.0

(22) Date of filing: 01.09.83

(51) Int. Cl.³: C 07 D 405/04, C 07 D 317/66, C 07 D 401/04, C 07 D 207/27, A 61 K 31/40

(30) Priority: 02.09.82 GB 8225010

(43) Date of publication of application: 02.05.84 Bulletin 84/18

(84) Designated Contracting States: BE CH DE FR GB IT LI NL SE

(71) Applicant: THE WELLCOME FOUNDATION LIMITED, 183-193 Euston Road, London NW1 2BP (GB)

(72) Inventor: Hodgson, Gordon Lewis, 115 Radcliffe Circle, Durham North Carolina 27713 (US)
Inventor: Harfenist, Morton, Rt. 6, Box 405, Chapel Hill North Carolina 27514 (US)

(74) Representative: Berg, Wilhelm, Dr. et al, Dr. Berg, Dipl.-Ing. Stapf, Dipl.-Ing. Schwabe, Dr. Dr. Sandmair Mauerkircherstrasse 45, D-8000 München 80 (DE)

## (54) Pharmacologically active lactam derivatives.

(57) Compounds of formula (I) and acid addition salts thereof are of use in medicine in the treatment of prophylaxis of pain, inflammation or fever.

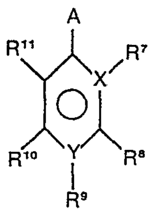

(I)

The compounds of formula (I) may be prepared by methods analogues to those known in the art. When used in medicine, the compounds of formula (I) may be administered as the compound alone or as a pharmaceutical formulation together with a pharmaceutically acceptable carrier.



ACTORUM AG

<u>CHEMICAL COMPOUNDS</u>

This invention relates to compounds useful in medicine, to their synthesis, pharmaceutical formulations thereof, the preparation of such formulations and to their use in the treatment or prophylaxis of pain, inflammation or fever.

Accordingly, one aspect of the present invention relates to pharmaceutical formulations comprising compounds of formula (I) in association with pharmaceutically acceptable carries thereof,

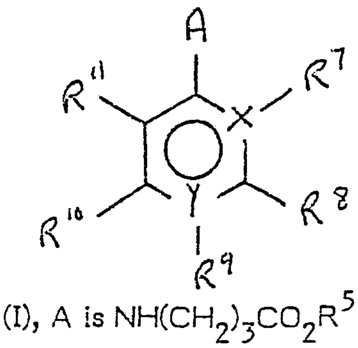

(I)

wherein, in formula (I), A is $NH(CH_2)_3CO_2R^5$ or

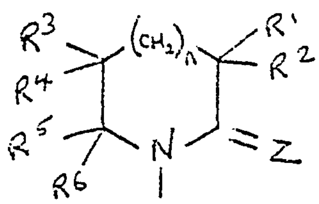

and,
$R^1$ and $R^2$ are the same or different and each may be hydrogen, alkyl, alkenyl, alkoxy, $CO_2$-alkyl, hydroxy and $CO_2H$, $R^3$ and $R^4$ are the same or different and each may be hydrogen, alkyl, alkenyl, aralkyl, hydroxy, alkoxy, $CO_2H$, $CO_2$ alkyl or halogen;

$R^5$ and $R^6$ are the same or different and each may be hydrogen or alkyl;

$R^7$ is hydrogen, alkyl, hydroxy, alkoxy, O-alkenyl, O-alkynyl, alkyl substituted by halogen, amino, nitro, $OCH_2$ $CO_2H$ or halogen;

$R^8$ is hydrogen, hydroxy, alkyl, alkenyl, aralkyl, O-alkyl, alkyl substituted by halogen, halogen, O-acyl, nitro, amino, CH-CH - $CH_2$, $CH_2$ CH $(OH)CH_2Cl$ or 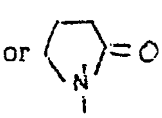 $CO_2H$;

$R^9$ is hydrogen, alkyl, O-alkyl, O-alkenyl, hydroxy, amino, cyano, acylamino, O-

acyl or $CO_2H$;

$R^8$ and $R^9$ together may be, -CH=N-NH-, $-(CH_2)_3$, $-O(CH_2)_2O-$, $-OCH(CH_3)O-$, -$OCF_2O$- $-OC(CH_3)_2O-$, $-OCH_2O$, $-OCH(CO_2Et)O-$, $-OCH(CO_2H)O-$, or - OC -O-;

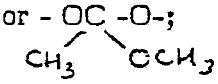

$R^{10}$ is hydrogen, alkyl, alkyl substituted by halogen, O-alkyl, or halogen;

$R^{11}$ is hydrogen;

n is O or 1;

X is a nitrogen or a carbon atom, and Y is a nitrogen or carbon atom, provided that when one of X and Y is nitrogen the other is carbon; and

Z is oxgen or NH;

provided that when $R^5$, $R^6$. $R^7$, $R^{10}$ and $R^{11}$ are all hydrogen and $R^8$ and $R^9$ together are $-OCH_2O-$, $R^1$ to $R^4$ are not all hydrogen.

A further aspect of the present invention relates to pharmaceutical formulations containing compounds of formula (II), which are within the scope of formula (I):-

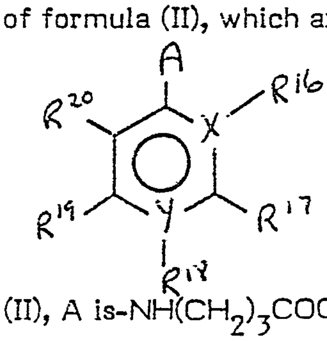

(II)

In compounds formula (II), A is-$NH(CH_2)_3COOH$, or,

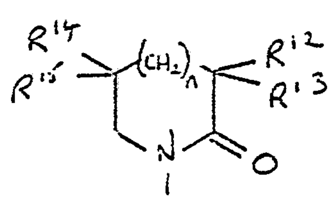

and, $R^{12}$ and $R^{13}$ are the same or different and each is hydrogen or alkyl; $R^{14}$ and $R^{15}$ are the same or different and each is hydrogen, halogen, or alkyl; $R^{16}$ is hydrogen, hydroxy, alkyloxy, O-alkenyl or O-alkynyl;

$R^{17}$ is hydrogen, hydroxy, alkyl, O-alkyl, alkyl substituted with halogen, halogen, or alkenyl; $R^{18}$ is hydrogen, alkyl, O-alkyl, or O-alkenyl; $R^{19}$ is hydrogen, alkyl or O-alkyl; $R^{20}$ is hydrogen; $R^{17}$ and $R^{18}$ together may be -CH=N-NH-, $-(CH_2)_3-$, $-O-(CH_2)_2O$, $-O\ CH(CH_3)-O-$, $OCF_2O-$, $-OC(CH_3)_2O-$, $-OCH_2O-$, n is O or 1; X is a nitrogen or carbon atom, and Y is a nitrogen or carbon atom, provided that, when one of X and Y is nitrogen, then the other is carbon;

with the proviso that when $R^{16}$, $R^{19}$ and $R^{20}$ are all hydrogen and $R^{17}$ and $R^{18}$ together are $-OCH_2O-$, $R^{12}$ to $R^{15}$ are not all hydrogen.

A yet further aspect of the present invention, relates to pharmaceutical formualtions comprising compounds of formula (III).

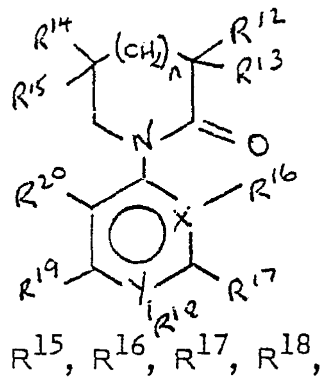

(III)

wherein, $R^{12}$, $R^{13}$, $R^{14}$, $R^{15}$, $R^{16}$, $R^{17}$, $R^{18}$, $R^{19}$, $R^{20}$, X, Y and n, are as hereinbefore defined in association with a pharmaceutically acceptable carrier thereof and with the proviso that when $R^{16}$, $R^{19}$ and $R^{20}$ are all hydrogen, and $R^{17}$ and $R^{18}$ together are $-OCH_2O-$, $R^{12}$ to $R^{15}$ are not all hydrogen.

Preferably, and the compound of formula (III) is selected from,

1-(1,3-benzodioxol-5-yl)-4-methyl-pyrrolidin-2-one
1-(4-allyloxyphenyl)-pyrrolidin-2-one
trans-1-(1,3-benzodioxol-5-yl)-3,4-dimethyl-pyrrolidin-2-one
1-(2,5-dimethoxyphenyl)-pyrrolidin-2-one
1-(1H-indazol-5-yl)-pyrrolidin-2-one
1-(5-indanyl)-pyrollidin-2-one
1-(1,3-benzodioxol-5-yl)-2-piperidin-2-one
1-(3,5-dimethoxyphenyl)-pyrrolidin-2-one
1-(2,2-dimethyl-1,3-benzodioxol-5-yl)-pyrrolidin-2-one
1-(2,2-difluoro-1,3-benzodioxol-5-yl)-pyrrolidin-2-one
1-(2-methyl-1,3-benzodioxol-5-yl)-pyrrolidin-2-one
1-(4-hydroxy-3-methoxyphenyl)-pyrrolidin-2-one

1-(3-hydroxy-4-methoxyphenyl)-pyrrolidino-2-one
1-(2-methyl-4-pyridyl)-pyrrolidin-2-one
1-(4-pyridyl)-piperidin-2-one
1-(3,4-diethoxyphenyl)-pyrrolidin-2-one
1-(4-pyridyl)-pyrrolidin-2-one
1-(3-ethylphenyl)pyrrolidin-2-one
1-(2-pyridyl)-pyrrolidin-2-one
3-methyl-1-phenyl-pyrrolidin-2-one
1-(3,5-dimethyl-2-pyridyl)pyrrolidin-2-one
1-(3-methyl-2-pyridyl)pyrrolidin-2-one
1-(2,3-dihydro-1,4-benzodioxin-6-yl)pyrrolidin-2-one
1-(1,3-benzodioxol-5-yl)-3-methyl-pyrrolidin-2-one

1-(-2-allyloxyphenyl)-pyrrolidin-2-one
1-(2-hydroxy-3-allylphenyl)-pyrrolidin-2-one
1-(2-prop-2-yn-yloxyphenyl)-pyrrolidin-2-one
1-(2-but-2-yn-yloxylphenyl)-pyrrolidin-2-one
1-(2-(1-methyl-prop-2-yn-yl)oxyphenyl)-pyrrolidin-2-one
1-(4-methylphenyl)-pyrrolidin-2-one
1-(4-fluorophenyl)-pyrrolidin-2-one
1-(4-trifluoromethylphenyl)-pyrrolidin-2-one
1-(3-methylphenyl)-pyrrolidin-2-one
1-phenyl-piperidin-2-one
1-(3-methoxyphenyl)-pyrrolidin-2-one
1-(3-chlorophenyl)-pyrrolidin-2-one
1-(3,4-dimethoxyphenyl)pyrrolidin-2-one

In a still further, aspect of the present invention there is provided within the scope of formula (I) compounds of formula (IA), which are believed to be novel.

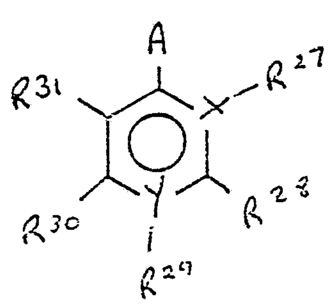

(IA)

In formula (IA), A is $NH(CH_2)_3CO_2R^{25}$ or 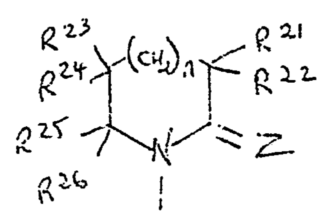

$R^{21}$, $R^{22}$, $R^{23}$ and $R^{24}$ are the same or different and each may be hydrogen, alkyl, alkenyl, alkoxy or $CO_2$ alkyl.

$R^{25}$ and $R^{26}$ are the same or different and each may be hydrogen or alkyl.

$R^{27}$ is hydrogen, alkyl, hydroxy, alkoxy, O-alkenyl, or O-alkynyl.

$R^{28}$ is hydrogen, hydroxy alkenyl, alkoxy, alkyl, O-acyl, 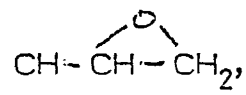, $CH_2CH(OH)CH_2Cl$ or 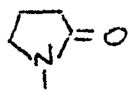

$R^{29}$ is hydrogen , O-alkenyl, hydroxy, O-acyl or alkoxy; or

$R^{28}$ and $R^{29}$ together may be -CH=N-NH-, $-(CH_2)_3-$, $-O(CH_2)_2O-$, $-OCH(CH_3)O-$, $-OCF_2O-$, $-OC(CH_3)_2O-$, $-OCH_2O$;

$R^{30}$ is hydrogen, alkyl, or O-alkyl;

$R^{31}$ is hydrogen;

n is O or 1,

X is a nitrogen or a carbon atom, and Y is a nitrogen or carbon atom, provided that when one of X and Y is nitrogen the other is carbon;

Z is oxygen or NH,
with the proviso that when $R^{25}$, $R^{26}$, $R^{27}$, $R^{30}$ and $R^{31}$ are all hydrogen and $R^{28}$ and $R^{29}$ together are $OCH_2O$, $R^{21}$- $R^{24}$ are not all hydrogen.

Preferably the compound of formula(IA) is selected from,

1-(1,3-benzodioxol-5-yl)-4-methyl-pyrrolidin-2-one
1-(4-allyloxyphenyl)-pyrrolidin-2-one
1-(1,3-benzodioxol-5-yl)-3,3-dimethyl-pyrrolidin-2-one
trans-1-(1,3-benzodioxol-5-yl)-3,4-dimethyl-pyrrolidin-2-one
1-(2,5-dimethoxyphenyl)-pyrrolidin-2-one
1-(1H-indazol-5-yl)-pyrrolidin-2-one

1-(5-indanyl)-pyrrolidin-2-one
1-(1,3-benzodioxol-5-yl)-piperidin-2-one
1-(3,5-dimethoxyphenyl)-pyrrolidin-2-one
1-(2,2-dimethyl-1,3-benzodioxol-5-yl)-pyrrolidin-2-one
1-(2,2-difluoro-1,3-benzodioxol-5-yl)-pyrrolidin-2-one
1-(2-methyl-1,3-benzodioxol-5-yl)-pyrrolidin-2-one
1-(4-hydroxy-3-methoxyphenyl)-pyrrolidin-2-one
1-(3-hydroxy-4-methoxyphenyl)-pyrolidin-2-one
1-(2-methyl-4-pyridyl)-pyrrolidin-2-one
1-(4-pyridyl)-piperidin-2-one
1-(3,4-diethoxyphenyl)-pyrrolidin-2-one
1-(4-pyridyl)-pyrrolidin-2-one
1-(3-ethylphenyl)-pyrrolidin-2-one
1-(2-pyridyl)-pyrrolidin-2-one
3-methyl-1-phenyl-pyrrolidin-2-one
1-(3,5-dimethyl-2-pyridyl)pyrrolidin-2-one
1-(3-methyl-2-pyridyl)pyrrolidin-2-one
1-(2,3-dihydro-1,4-benzodioxin-6-yl)-pyrrolidin-2-one
1-(1,3-benzodioxol-5-yl)-3-methyl-pyrrolidin-2-one
1-(2-allyloxyphenyl)-pyrrolidin-2-one
1-(2-hydroxy-3-allylphenyl)-pyrrolidin-2-one
1-(2-prop-1-yn-yloxyphenyl)-pyrrolidin-2-one
1-(2-but-2-yn-yloxylphenyl)-pyrrolidin-2-one
1-(2-(1-methyl-prop-2-yn-yl)oxyphenyl)-pyrrolidin-2-one
4-(1,3-benzodioxol-5-ylamino)butyric acid.

3-allyl-1-(1,3-benzodioxol-5-yl)-pyrrolidin-2-one
1-(1,3-benzodioxol-5-yl)-3-ethyl-pyrrolidin-2-one
2-allyl-4-(2-oxo-1-pyrrolidinyl)-phenyl acetate
1-(4-acetoxyphenyl)-5-methyl-pyrrolidin-2-one
1-(4-hydroxyphenyl)-5-methyl-pyrrolidin-2-one
1-(4-acetoxy-3-allyl phenyl)-pyrrolidin-2-one
1-(4-acetoxy-3-(2-oxiranyl methyl)phenyl)-pyrrolidin-2-one
1-[3-(3-chloro-2-hydroxylpropyl)-4-hydroxyphenyl]-pyrrolidin-2-one
2-(2-oxy-1-pyrrolidinyl)phenoxyacetic acid
Methyl 1-(1,3-benzodioxol-5-yl)-5-oxo-3-pyrrolidine carboxylate
5-(2-oxo-1-pyrrolidinyl)-1,3-benzodioxol-2-carboxylic acid
Ethyl 5-(2-oxo-1-pyrrolidinyl)-1,3-benzodioxol-2-carboxylate
Ethyl 1-(1,3-benzodioxol-5-yl)-5-oxo-3-pyrrolidine carboxylate

1-(3,4-diethoxyphenyl)-pyrrolidin-2-one
1-(4-propionyloxyphenyl)-pyrrolidin-2-one
1-(4-acetoxy-3-(2-oxiranylmethyl)phenyl)-pyrrolidin-2-one
1-[3-(3-chloro-2-hydroxypropyl)-4-hydroxyphenyl]-pyrrolidin-2-one
1-(1,3-benzodioxol-5-yl)-4-hydroxymethyl-pyrrolidin-2-one

In this specification the term "alkyl" either alone or as part of another group eg. O-alkyl, shall be taken to refer to an alkyl group having from 1 to 6 carbon atoms, preferably 1 to 4 carbon atoms.

The term "alkenyl", either alone or as part of another group shall be taken to refer to an alkenyl group having from 3 to 6 carbon atoms.

The term "alkynyl", either alone or as part of another group shall be taken to refer to an alkynyl group if from 3 to 6 carbon atoms, preferably 4 to 6 carbon atoms.

The term "aralkyl" shall be taken to refer to an alkyl group substituted by one or more aryl groups. The term "aryl" shall be taken to refer to an aromatic ring system of from 5 to 10 atoms selected from carbon and nitrogen, which may optionally be substituted by one or more valuable substituents. Examples of aryl groups include phenyl, quinolyl, pyridyl and naphthyl. "Suitable substituents " will be appreciated by those skilled in the art.

The compounds of formual (I) have been found to have mild to moderately strong analgesic activity. As analgesic agents compounds of formula (I) are like morphine and codeine but superior to aspirin or acetaminophen as shown in the trypsin assay and the hot plate assay. However, the analgesic mode of action of the compounds of formula (I) is believed to be unlike that of morphine or codeine since their analgesic activity is not inhibited by naloxone, and they do not bind to the morphine receptor. Thus compounds of formula (I) are considered non-narcotic. The duration of analgesic action is significantly greater for compounds of formula (I) than for codeine or morphine.

Compounds of formula (I) have also been found to have potent, long-lasting acute anti-inflammatory activity in the rat as shown in the carrageenan pleurisy assay (Vinegar et al., Proc. Soc. Exp. Biol. Med. 151, 556, (1976)). Compounds of formula (I) resemble acetaminophen in their acute anti-inflammatory action but may have been found to be more potent and to have a longer lasting anti-inflammatory effect at comparable dose levels.

Compounds of formula (I), like acetaminophen, have also been found to have antipyretic and hypothermic activity as shown by the yeast-induced hyperthermia assay in the rat (Khalili-Varasteh et al., Arch. Int. Pharmacodyn., 219, 149-159 (1976)). This is to say, the compounds of formula (I) combat fever in the rat as does aspirin and acetominophen.

Compounds of formula (I) may be used in the relief, treatment or prophylaxis of pain (moderate to severe), inflammation or fever, in a mammal, including man, such as: that resulting from headache, toothache, pain following general dental procedures, oral and general surgery, dysmenorrhea, myalgia, pain of unresectable cancer, joint and peripheral nerve disorders, rheumatoid arthritis, rheumatoid spondylitis, osteoarthritis, gouty arthritis and other arthritic conditions, pyresis and other conditions associated with pain, inflammation and fever.

The amount of the active compound, i.e. compound of formula (I), required for use in the above conditions will, of course, vary both with the route of administration, the condition under treatment, and the mammal undergoing treatment, but is ultimately at the discretion of the physician. However, a suitable analgesic, anti-inflammatory and/or anti-pyretic dose of the active compound for a mammal is in the range of from 3 to 120 mg per kilogram bodyweight per day; a typical dose for a human recipient being 15 mg/kg bodyweight per day.

The desired dose is preferably presented as a divided dose in the range of from two to four subdoses administered at appropriate intervals throughout the day. Thus where three sub-doses are employed each will lie in the range of from 1 to 20 mg (base)/kg body weight, a typical dose for a human recipient being 3 mg (base)/kg body weight.

While it is possible for the active compound to be administered alone as the raw chemical, it is preferable to present the active compound as a pharmaceutical formulation. Formulations of the present invention, both for veterinary and for human medical use, comprise the active compound together with one or more pharmaceutically acceptable carriers thereof and optionally any other therapeutic ingredients. The carrier(s) must be pharmaceutically acceptable in the sense of being compatible with the other ingredients of the formulation and not deleterious to the recipient thereof. The other therapeutic ingredient(s) may include other analgesics (such as codeine) anti-inflammatories or anti-pyretics.

The formulations include those suitable for oral, rectal or parenteral (including subcutaneous, intramuscular and intravenous) administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier or a finely divided solid carrier or both and then, if necessary, shaping the product into the desired formulation.

Formulations of the present invention suitable for oral administration may be presented as discrete units such as capsules, cachets, tablets or lozenges, each containing a predetermined amount of the active compound; as a powder or granules; or a suspension in an aqueous liquid or non-aqueous liquid such as a syrup, an elixir, an emulsion or a draught. The active compound may also be presented as a bolus, electuary or paste.

A tablet may be made by compression or moulding, optionally with one or more accessory ingredients. Compressed tablets may be prepared by compressing, in a suitable machine, the active compound being in a freeflowing form such as a powder or granules, optionally mixed with a binder, lubricant, inert diluent,surface active or dispersing agent. Moulded tablets may be made by moulding in a suitable machine, comprising a mixture of the powdered active compound with any suitable carrier.

A syrup may be made by adding the active compound to a concentrated, aqueous solution of a sugar, for example sucrose, to which may also be added any accessory ingredient. Such accessory ingredient(s) may include flavourings, an agent to retard crystallization of the sugar or an agent to increase the solubility of any other ingredient, such as polyhydric alcohol for example glycerol or sorbitol.

Formulations for rectal administration may be presented as a suppository with a usual carrier such as cocoa butter. Formulations suitable for parenteral administration conveniently comprise a sterile aqueous preparation of the active compound which is preferably isotonic with the blood of the recipient.

In addition to the aforementioned ingredients, the formulations of this invention may further include one or more accessory ingredient(s) selected from diluents, buffers, flavouring agents, binders, surface active agents, thickeners, lubricants, preservatives (including antioxidants) and the like.

Compounds of formula (I) and (II) may be prepared by any method known in the art for the preparation of compounds of analogous structure.

For example,

(1) One method for preparing compounds of formula (I) wherein A is 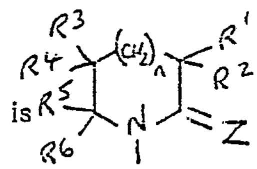

comprises cyclisation, as hereinafter described, of a compound of formula (IV) or a compound of formula (V):

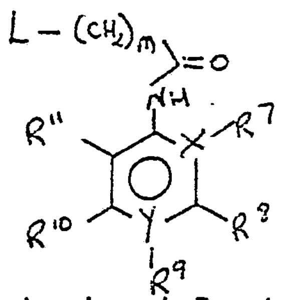 (IV)

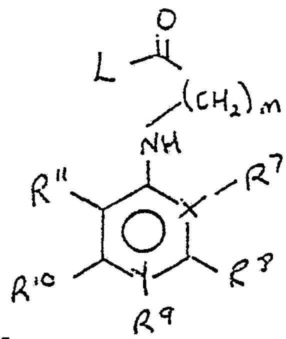 (V)

wherein m is 3 or 4, $R^7$, $R^8$, $R^9$, $R^{10}$ and $R^{11}$ are as hereinbefore defined and wherein L is a standard leaving group (J.March, Advanced Organic Chemistry, 2nd Ed., page 187, New York (1977)) such as halide for example chloride or bromide, hydroxide, $-OR^{32}$, imidazolyl, sulphoxonium or tosyl; and $R^{32}$ is hydrogen or alkyl, preferably ethyl. Preferred compounds of formula (IV) are those wherein the leaving group is a halide (such as chloride or bromide), hydroxide or tosyloxy, and preferred compounds of formula (V) are those wherein the leaving group is $-OR^{32}$ as defined.

A particularly preferred method comprises cyclisation of a compound of formula (IV) as hereinbefore defined, especially wherein L is chloride.

Cyclisation may be effected at room temperature or with heating for example at a temperature of $155^{\circ}$-$220^{\circ}C$, optionally in an oxygen-free atmosphere for example in nitrogen, optionally in an inert solvent such as tetrahydrofuran, dichloromethane, diethyl ether, <u>tert</u>-butanol, xylenes, or toluene, and optionally with a catalyst.

The catalyst chosen will depend on the compound of formula (III) or (IV) to be cyclised, for example, where the reaction involves elimination of an acid such as hydrochloric, a basic catalyst may be used with or without a solvent such as water or an alcohol such as butanol optionally, but preferably, in the presence of a phase transfer catalyst such as triethylbenzyl ammonium chloride with or without a solvent such as dichloromethane, diethyl ether, xylenes or toluene, but preferably dichloromethane. Examples of suitable basic catalysts are: an alkali metal hydride, hydroxide or alkoxide such as potassium or sodium hydride, potassium or sodium hydroxide, potassium tert-butoxide or lithium di-isopropylamide, triethylamine, and pyridine. The most preferred method of cyclisation is effected by using aqueous sodium hydroxide in the presence of triethylbenzyl ammonium chloride at room temperature.

Where L is a slow or poor leaving group cyclisation may take place by conversion in situ to a further or better leaving group. For example where L is hydroxide, tosyl chloride may be present in the reaction mixture in order that the tosyloxy group (a better leaving group) is substituted for the hydroxide group thereby causing cyclisation to proceed faster and more completely.

Similarly, compounds of formula (IA) wherein A is 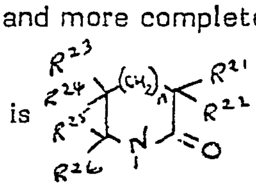 may be prepared by cyclisation of a corresponding compound of formula (IVa) or a compound of formula (Va),

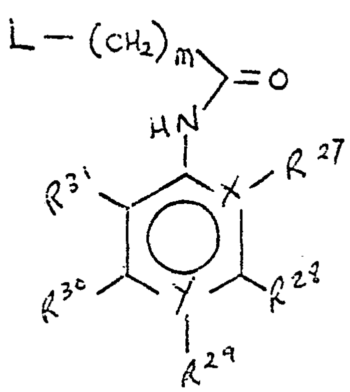 (IVa)

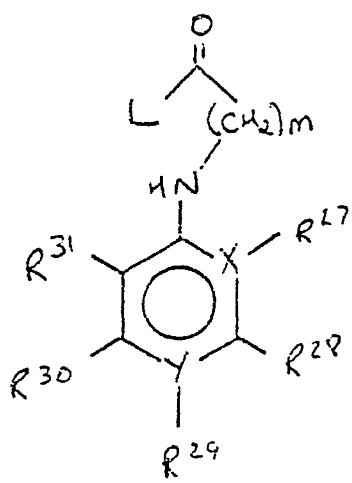 (Va)

wherein $R^{27}$ to $R^{31}$, X, Y m and L are hereinbefore defined.

(2) One method for preparing compounds of formula (III) wherein n=O, and one or more of $R^{12}$ $R^{15}$, is an alkyl group of 1-4 carbon atoms, comprises reaction of a corresponding 1-substituted pyrrolidin-2-one of formula (VI), wherein $R^{16}$, $R^{17}$, $R^{18}$, $R^{19}$, $R^{20}$, X and Y, are as hereinbefore defined, with a base, and an alkyl donor. Examples of suitable bases are : alkali metal hydride, hydroxide or alkoxide, such as potassium or sodium hydride, potassium or sodium hydroxide, potassium t-butoxide or lithium di-isopropylamide. Suitable alkyl donors include alkyl halides. The most preferred method uses lithium di-isopropylamide and the appropriate alkyl iodide.

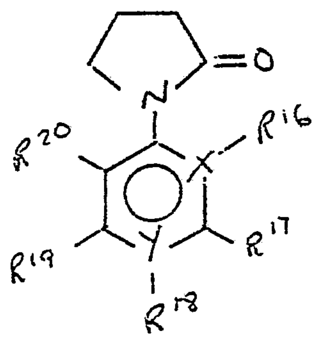

(VI)

(3) One method for preparing compounds of formula (III) wherein n is O and one of $R^{16}$, or $R^{18}$, is O-alkenyl or O-alkynyl, comprises reaction of the corresponding compounds of formula (VII), and (VIII)

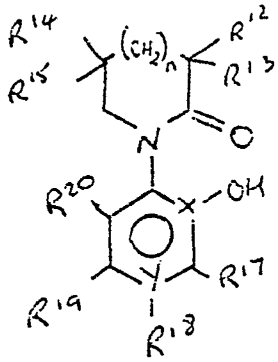

(VII)

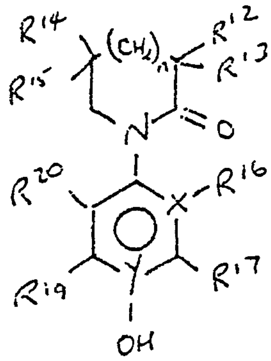

(VIII)

wherein $R^{12}$, $R^{13}$, $R^{14}$, $R^{15}$, $R^{17}$, $R^{18}$, $R^{19}$, $R^{20}$, n , X and Y are as defined herein above, provided that in (VII) $R^{18}$ is hydrogen and X is carbon and in (VIII) $R^{16}$ is H and Y is carbon; with a base an d an alkenyl or alkynyl donor. Suitable bases include those defined in (2) above, and alkali metal alkoxides, for example sodium methoxide. Suitable donors include alkenyl halides and alkynyl halides for example allyl bromide, and propargyl bromide.

(4) A further method for the preparation of compounds of formula (III) comprises hydrolysis of compounds of formula (IX) wherein n, X, Y and $R^{16}$ to

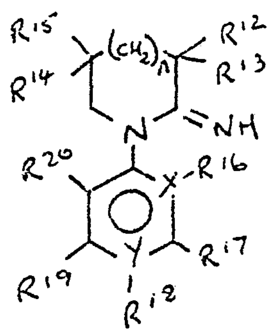

(IX)

The hydrolysis may be effected by standard hydrolysing agents known to those skilled in the art, for example, by adding a few drops of water or dilute aqueous acid to the compounds.

The compounds of formula (IX) may themselves be prepared according to the method described by Kwok et al in J.Org. Chem. (1967) 32, 738.

(5) A further method for the preparation of compounds of formula (III) wherein n is O comprises a displacement reaction between a compound of formula (X), and the pyrrolidinone anion formula (XI)

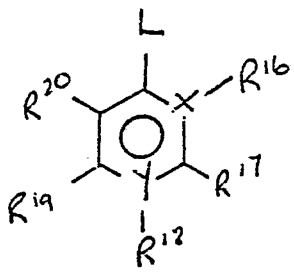

(X)

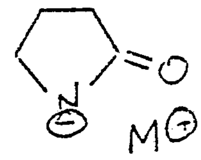

(XI)

wherein L is a standard leaving group such as those hereinbefore defined, $M^+$ is an alkali metal or alkaline earth metal cation such as $Na^+$, and $R^{16}$ to $R^{20}$, X and Y are as hereinbefore defined.

(6) Another method for the preparation of compounds of formula (III) comprises reacting compounds of formula (XII) wherein X, Y and $R^{16}$ to $R^{20}$ are as hereinbefore defined with $\gamma$-butyrolactone, or 3-methyl-$\gamma$-butyrolactone.

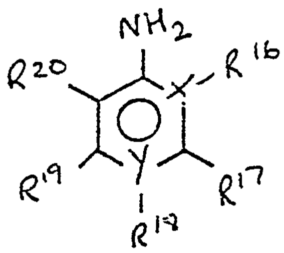

(XII)

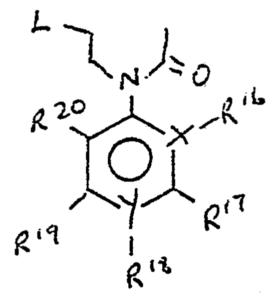

(XIII)

(7) Yet another preparation of compounds of formula (III) consists of subjecting a compound of formula (XIII) (wherein L, X, Y and $R^{16}$ to $R^{20}$ are as hereinbefore defined) to ring closure conditions similar to those described in method (1) above. Compounds of formula (XIII) may be prepared by reacting compounds of formula (XII) with a compound of formula (XIV), wherein $L^1$ and $L^2$ may be the same or different and each is L as defined in method (1) above,

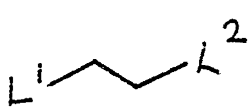

(XIV)

followed by acylation of the nitrogen atom by standard methods.

(8) The compound of formula (II) wherein $R^{12}$, $R^{13}$, $R^{14}$, $R^{15}$, $R^{18}$, $R^{19}$ and $R^{20}$ are all hydrogen, $R^{16}$ is hydroxy, $R^{17}$ is allyl, n is O, and X and Y are both carbon, may also be prepared by Claisen rearrangement of a compound of formula (XV)

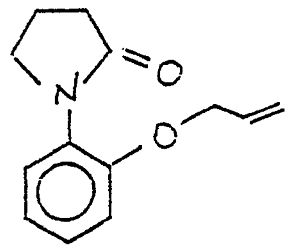

(XV)

(9) A further method for the preparation of compounds of formula (III) wherein $R^{14}$ or $R^{15}$ is $C_{1-4}$alkyl, comprises reaction of a compound of formula (XVI), wherein P is $C_{1-4}$alkyl, and X, Y and $R^{16}$ to $R^{20}$ are as hereinbefore defined for formula (III) with a suitable dialkyl sulphoxonium methylide, for example where $R^{14}$ or $R^{15}$ is methyl, by reaction with dimethyl sulphoxonium methylide.

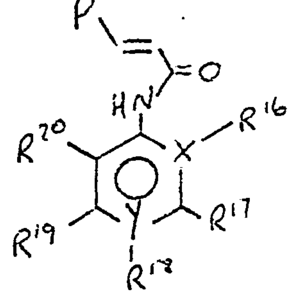

(XVI)

(10) A yet further method, for the preparation of compounds of formula (III) comprises reduction of the corresponding oxidised precursor of a compound of formula (III), for example, reduction of compounds of formula (XVII) wherein X, Y, n, and $R^{12}$ to $R^{20}$ are all as hereinbefore defined for formula (III).

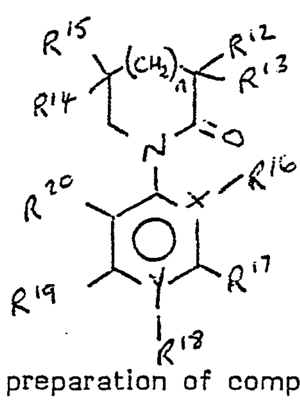

(XVII)

(11) A method for the preparation of compounds of formula (I) wherein A is $NH(CH_2)_3CO_2H$ comprises base catalysed ring opening of the corresponding pyrrolidinone derivatives of formula (VI). Alternatively, compounds of formula (I) wherein A is $NH(CH_2)_3CO_2H$ may be prepared by the reaction of compounds of formula (XII) with a suitable reagent, for example, $Br(CH_2)_3CO_2C_2H_5$ (followed by hydrolysis) of the intermediate so formed.

The compounds of formula (IV) or (V) may themseleves be prepared by analogous methods known to those skilled in the art, for example, by reacting the compound of formula (XII) or a salt thereof such as an acid addition salt thereof, for example the hydrochloride, or an alkali metal or alkaline earth metal salt thereof, for example the lithium salt, with an internal ester, acid halide for example acid chloride, or acid anhydride. For example, the compound of formula (XII) may be reacted with $Cl-(CH_2)_3-COCl$ to produce a compound of formula (IV) wherein L is chloride in the presence of triethylamine in dimethoxyethane or dichloromethane, and wherein $R^{16}$ to $R^{20}$ are as hereinbefore defined.

The reaction may be carried out under the same or similar conditions as described hereinbefore, for cyclisation, since the compound of formula (IV) or (V) need not be isolated but may be cyclised in situ, for example, by a method analogous to those described by A.Pernot and A Willemont in Memoires Presentes a La Soc. Chem. 324. (1953); W.R.Schleigh, A.Catala and F.D. Popp in J. Het. Chem., 2, 379 (1965) or I. Badilescu in Tetrahedron, 26, 4207, 1970.

The following Examples are provided by the way of illustration of the present invention and should in no way be construed as a limitation thereof. All temperatures indicated are in degrees Celsius.

Example 1. Preparation of 1-(1,3-Benzodioxol-5-yl)-4-methyl-pyrrolidin-2-one

Example 1A: Preparation of N-(1,3-Benzodioxol-5-yl)-2-butenamide

Crotonyl chloride (14.0 ml, 0.15 mole) in tetrahydrofuran (20 ml) was added dropwise to a mixture of 3-4- methylenedioxyaniline (20 g, 0.15 mole) and triethylamine (20 ml, 0.15 mole) in tetrahydrofuran (30 ml) at $0^{o}C$ under an atmosphere of nitrogen. The reaction mixture was stirred at $0^{o}C$ for 1 hour, allowed to warm to room temperature and quenched with a portion of ethanol.The reaction mixture was filtered and solvent removed in vacuo to give a solid. This solid was washed with water and re-crystallised from dichloromethane to give N-(1,3-benzodioxol-5-yl)-2-butenamide (12g m.p. 143-144$^{o}$).

Example 1B. Preparation of 1-(1,3-Benzodioxol-5-yl)-4-methyl-pyrrolidin-2-one

N-(1,3-Benzodioxol-5-yl)-2-butenamide (5.6g, 0.027 mole) in tetrahydrofuran (120 ml) was added dropwise to a solution of dimethylsulphoxonium methylide (0.04 mole) /generated from trimethyl sulphoxonium iodide (9.0g, 0.041 mole) and potassium hydride (3.4g of 50% suspension, 0.07 mole) in tetrahydrofuran (20 ml) at $0^{0}$ under an atmosphere of nitrogen/, at $0^{0}$C, the mixture was allowed to warm to room temperature and then stirred for 12 hours. The mixture was quenched with a portion of ethanol, filtered, the solvent removed in vacuo to give a solid which was recrystallised from dichloromethane/ethylether/light petroleum to yield 1-(1,3-benzodioxol-5-yl)-4-methyl-pyrrolidin-2-one (4.9g m.p. 102-$105^{0}$).

Example 2: Preparation of 1-(4-allyloxyphenyl)-pyrrolidin-2-one

Sodium methoxide (12.3g, 0.23 mole) was added to a solution of 1-(4-hydroxyphenyl)-pyrrolidin-2-one (15g 0.084 mole) in anhydrous dimethyl sulphoxide (85 ml) under a nitrogen atmosphere, and the reaction mixture stirred for 30 mins at ambient temperature. The reaction mixture was then cooled to $0^{0}$C and allyl bromide (20.3g, 0.17 mole) added, dropwise. Water was then added, and the resulting solids isolated and washed with dilute sodium hydroxide solution. Recrystallisation from chloroform, and then ether/pentane, afforded 1-(4-allyloxyphenyl)-pyrrolidin -2-one (7.85g m.p. 100-$103^{0}$).

Example 2A : Preparation of 1-(4-propionyloxyphenyl)-pyrrolidin-2-one.

A procedure analogous to that of example 2 was used to prepare the title compound m.p. 79-$80^{0}$.

Example 3: Preparation of 1-(1,3-benzodioxol-5-yl)3,3-dimethyl-pyrrolidin-2-one

A mixture of 3,4-(methylenedioxy)aniline (200 g, 1.46 mole) and $\gamma$-butyrolactone (225ml) was heated (in a dry nitrogen atmostphere) with stirring in a $200^{0}$ oil bath for 2 days. Product was isolated from the reaction mixture by distillation under reduced pressure (bp $156^{0}$, 33μ).

Product was filtered through Silica Gel 60 (Trade Name) eluting with ethyl acetate. The eluant was concentrated and the crystalline product was collected and washed with diethyl either and petroleum ether affording 1-(1,3-benzodioxol-5-yl)-2-pyrrolidinone (153.4 g; 51%) mp 89-91$^{o}$ which was one spot on tlc analysis. 1-(1,3-Benzodioxol-5-yl)-pyrrolidin-2-one(3,0g, 0.015 mole) in anhydrous tetrahydrofuran (50 ml) was added dropwise to a solution of lithium di-iso-propylamide (0.015 mole), in tetrahydrofuran (50 ml) under a nitrogen atmosphere at -78$^{o}$. The reaction mixture was then stirred for 5 mins, methyl iodide (1 ml, 0.016 moles) added, and the reaction stirred for a further 15 mins

[N.B. At this point the reaction could be worked up to give an alternative synthesis of 1,(1,3-benzodioxol-5-yl)-3-methyl-pyrrolidin-2-one - see example 26/.Further lithium di-isopropylamide was generated in situ by addition of n-butyl lithium (9.5ml, 0.015 mole), and the reaction mixture stirred for a further 15 minutes before additional methyl iodide (1 ml, 0.016 mole) was added. The mixture was then allowed to slowly warm to ambient temperature, quenched with a portion of ethanol, and the solvent evaporated to give an oil. This oil was partitioned between water and diethyl ether, the layers separated and the organic phases dried (sodium sulphate), and solvent removed in vacuo to give a solid. Recrystallisation from dichloromethane/light petroleum afforded 1-(1,3-benzodioxol-5-yl)-3,3-dimethyl-pyrrolidin-2-one (2.1g m.p. 104-105$^{o}$C).

Example 3A : Preparation of 1-(1,3-Benzodioxol-5-yl)-3-ethyl pyrrolidin-2-one.

A procedure analogous to that of example 3 was used to prepare the title compound m.p. 83-86$^{o}$.

Example 3B : Preparation of 3-allyl-1-(1,3-benzodioxol-5-yl)- pyrrolidin-2-one.

A procedure analogous to that of example 3 was used to prepare the title compound m.p. 77-78$^{o}$.

Example 4 : Preparation of trans-1-(1,3-benzodioxol-5-yl)3,4-dimethyl-pyrrolidin-2-one

Using the procedure described in example 3, 1-(1,3-benzodioxol-5-yl)-4-methyl-pyrrolidin-2-one (prepared as described in example 1) was used to prepare trans-1-(1,3-benzodioxol-5-yl)-3,4-dimethyl-pyrrolidin-2-one (m.p. 88-90$^{o}$C).

Example 5 : Preparation of 4-(1,3-benzodioxol-5-yl)amino butyric acid

1-(1,3-Benzodioxol-5-yl)-pyrrolidin-2-one (10.3g 0.05 mole) was added to a solution of sodium hydroxide (10.2 g) in methanol (250 ml). The solution was heated under reflux in an atmosphere of nitrogen for 48 hours, an additional amount of sodium hydroxide (4.0 g) and water (1.0 ml) was added, and the methanol removed from the mixture by distillation. The residue was dissolved in water (150 ml) and heated under reflux for a further 4 hours.The mixture was then allowed to cool, and stirred at ambient temperature for 12 hours. Dichloromethane was added, the mixture acidified with hydrochloric acid, and then extracted with ethyl acetate. The combined organic phases were dried (sodium sulphate) and the solvent removed in vacuo to give, 4-(1,3-benzodioxol-5-yl)amino butyric acid (9.0g m.p. 99-101$^{o}$).

Example 6 : Preparation of 1-(2,5-dimethoxyphenyl)-pyrrolidin-2-one

4-Chlorobutyryl chloride (8.8 ml, 0.078 mole) in tetrahydrofuran (10 ml) was added dropwise to a cooled solution of 2,5-dimethoxyaniline (10 g, 0.065 mole) in tetrahydrofuran (75 ml) under an atmosphere of nitrogen. Triethylamine (11 ml, 0.078 mole) was then added dropwise and the solution allowed to warm toambient temperature and stirred for 18 hours. Filtration and evaporation of solvent gave the intermediate butyramide as an oil which was dissolved in dichloromethane and aqueous sodium hydroxide (30-50% w/v, 50 ml). A catalytic amount of benzyl triethylammonium chloride (0.7 g) was added and the resulting reaction mixture stirred for 2.5 hours at ambient temperature. Dichloromethane and water were then added, the organic phase separated and washed with brine, and then dried over sodium sulphate. Evaporation of solvent, followed by recrystallisation from dichloromethane/light petroleum, gave 1-(2,5-dimethoxyphenyl)-pyrrolidin-2-one (2.2 g, m.p. 45.5-50$^{o}$).

A procedure analogous to that described in Example 6, was used to prepare the following compounds:

Example 7: 1-(1H-indazol-5-yl)-pyrrolidin-2-one, m.p. 201-202$^{0}$C

Example 8: 1-(5-indanyl-5-yl)-pyrrolidin-2-one, m.p. 100-101$^{0}$C

Example 9: 1-(1,3-benzodioxol-5-yl)-piperidin-2-one, m.p. 111-113$^{0}$C

Example 10: 1-(3,5-dimethoxyphenyl)-pyrrolidin-2-one, m.p. 82-85$^{0}$C

Example 11: 1-(2,2-dimethyl-1,3-benzodioxol-5-yl)-pyrrolidin-2-one, m.p. 99-101$^{0}$C.

Example 12: 1-(2,2-difluoro-1,3-benzodioxol-5-yl)-pyrrolidin-2-one, m.p. 91-93$^{0}$C

Example 13: 1(2-methyl-1,3-benzodioxol-5-yl)pyrrolidin-2-one, m.p. 54-56$^{0}$C

Example 14: 1-(4-hydroxy-3-methoxyphenyl)-pyrrolidin-2-one, m.p. 145-146$^{0}$C

Example 15: 1-(3-hydroxy-4-methoxyphenyl)-pyrrolidin-2-one, m.p. 175-178$^{0}$C

Example 16: Preparation of 1-(2-methyl-4-pyridyl)-pyrrolidin-2-one

4-Chlorobutyryl chloride (6.0 ml, 0.054 mole) in anhydrous tetrahydrofuran (50 ml) was added, dropwise with stirring, to a solution of 4-amino-2-methyl pyridine (5.4 g, 0.05 mole) and lutidine (6.5 ml, 0.056 mole) in anhydrous tetrahydrofuran (50 ml), at 0$^{0}$C under an atmosphere of nitrogen. The solvent was removed and the residue partitioned between dichloromethane and water. The pH was adjusted to 12 with sodium hydroxide solution (6N), the organic layer separated and dried (sodium sulphate). Removal of solvent under reduced pressure afforded the crude butyramide.

A solution of this butyramide (9.4 g) in anhydrous tetrahydrofuran was added dropwise to a slurry of potassium hydride (2 g, 0.05 mole) in anhydrous tetrahydrofuran, at 20$^{0}$C with stirring.

After stirring for one hour, t-butanol was added (4 ml) and the product partitioned between dichloromethane and water. The organic layer was separated, dried (sodium sulphate) and concentrated in vacuo to give a solid, which was recrystallised first from toluene/pentane, and then from dichloromethane/diethyl ether/light petroleum, to yield 1-(2-methyl-4-pyridyl)-pyrrolidin-2-one (1.9 g, m.p. 94-96°C).

Example 17: 1-(4-pyridyl)-piperidin-2-one

A procedure analogous to that described in Example 16 above, was used to prepare 1-(4-pyridyl)-piperidin-2-one, m.p. 119-120°C.

Example 18: 1-(3,4-diethoxyphenyl)-pyrrolidin-2-one

A solution of 3,4-diethoxyaniline (24.5 g, 0.14 mole) in $\gamma$-butyrolactone (50 ml) was heated, with stirring under a nitrogen atmosphere in a 200°C oil bath for 6 days. Distillation of the resultant reaction mixture afforded a fraction of b.p. 165-170° at 14-16µ. Recrystallisation from dichloromethane/light petroleum, afforded 1-(3,4-diethoxyphenyl)-pyrrolidin-2-one (21.2 g, m.p. 73-75°C).

A procedure analogous to that described in Example 18 was used to prepare the following compounds:-

Example 19: 1-(4-pyridyl)-pyrrolidin-2-one, m.p. 137-138°C

Example 20: 1-(3-ethylphenyl)-pyrrolidin-2-one, m.p. 37-39°C

Example 21: 1-(2-pyridyl)-pyrrolidin-2-one, m.p. 47-48.25°C

Example 22: 3-methyl-1-phenyl-pyrrolidin-2-one, m.p. 91-92°C

Example 23: 1-(3,5-dimethyl-2-pyridyl)-pyrrolidin-2-one, m.p. 57-57.5°C

Example 24: 1-(3-methyl-2-pyridyl)-pyrrolidin-2-one, m.p. 64-65°C

Example 25: 1-(2,3-dihydro-1,4-benzodioxon-6-yl)-pyrrolidin-2-one, m.p. 89-92°C

Example 26: 1-(1,3-benzodioxol-5-yl)-3-methyl-pyrrolidin-2-one, m.p. 76-78$^{0}$C

Example 27 : 1-(2-(prop-1-yn-yloxy)phenyl)-pyrrolidin-2-one

A mixture of 2-hydroxyphenyl-pyrrolidin-2-one (17.88 g, 0.1 mole), potassium tert-butoxide (12.58 mg, 0.11 mole) in tert-butanol was stirred for 5 mins under an atmosphere of nitrogen. Propargyl bromide (21.9g, 0.184 mole) was added, slowly with stirring, and the reaction then allowed to stand for 15 mins. The mixture was then heated under reflux overnight, allowed to cool, filtered, and the solid washed repeadtedley with acetone. The combined initial, and acetone filtrates were evaporated on a stream both at water pump pressure to leave an oily residue. Crystallisation of the oil was achieved by adding hot ethyl acetate followed by enough cold hexane to make the solution turbid, to yield the 1-(2-prop-1-yn-yloxyphenyl)-pyrrolidin-2-one (11.15 g, m.p. 115.5-117.3$^{0}$C).

Example 28: Preparation of 1-(2-allyloxyphenyl)-pyrrolidin-2-one

2-Hydroxyphenyl-pyrrolidin-2-one (17.7 g, 0.1 mole), allyl bromide (25.5 ml, 0.1 mole), and anhydrous potassium carbonate (70 g, 0.5 mole) were dissolved in acetone (150 ml), and the reaction mixture stirred vigourously and heated on a steam bath for 43 hours. The reaction mixture was then allowed to cool, and the solids filtered off and washed thoroughly with acetone. Evaporation of the combined filtrates gave an oil. This oil was dissolved in ether, and extracted with aqueous sodium hydroxide solution to remove any phenolic starting materials. The organic layer was dried (sodium sulphate), and the solvent removed to yield an oil which slowly crystallised. Recrystallised from ether afforded 1-(2-allyloxyphenyl)-pyrrolidin-2-one (15.3 g, m.p. 40.8-41.30$^{0}$C).

Example 29 : Preparation of 1-(2-hydroxy-3-allylphenyl)-pyrrolidin-2-one

1-(2-Prop-2-en-yloxyphenyl)-pyrrolidin-2-one (2.03 g) in N[N-dimethylaniline (50 ml) was heated under reflux overnight in an atmosphere of carbon dioxide. The product was partitioned between dichloromethane and hydrochloric acid (1N)with first, 1N aqueous sodium hydroxide (100 ml) and then 5N aqueous sodium hydroxide (50 ml). Acidification of the cooled first extract gave an oil which crystallised on standing. Recrystallisation from ether-hexane gave 1-(2-hydroxy-3-allylphenyl)-pyrolidin-2-one (0.46 g m.p. 83.3-84.9$^{0}$C).

Example 29(a) : Preparation of 2-Allyl-4-(2-oxo-1-pyrrolidinyl)-phenyl acetate

A procedure analogous to that of example 29 followed by acylation was used to prepare the title compound m.p. 48-50°.

Example 30 : 1-(2-(but-2-yn-yloxy)phenyl)-pyrrolin-2-one

2-Hydroxyphenyl-pyrrolidin-2-one (17.8 g), potassium *tert*-butoxide (13.4 g), but-2-yn-yl chloride (13.3 g) and sodium iodide (1.10 g) in *t*-butanol (100 ml) were heated under reflux for 6.5 hours. The reaction mixture was allowed to cool, diluted with ethanol, filtered, and the filtrates evaporated to give an oil. The oil was re-dissolved in ether, washed with aqueous sodium hydroxide, the ether layer then dried (sodium sulphate) and evaporated *in vacuo* to give an oil which was crystallised from ethyl acetate/hexane. A further recrystallisation using the same solvent gave, 1-(but-2-yn-yloxyphenyl)-pyrrolidin-2-one (12.9, m.p. 82.8-83.4°).

Example 31 : 1-(2-(1-methyl-prop-2-yn-yl)oxyphenyl)-pyrrolidin-2-one

A method analogous to that described in example 30, was used to prepare 1-(2-(1-methyl-prop-2-yn-yl)oxyphenyl)-pyrrolidin-2-one, 36.9 g, m.p. 91.6-93°.

Example 32 : Preparation of 1-(4-hydroxyphenyl)-5-methyl-pyrrolidin-2-one

4-Aminoaniline (25 g, 229 mmole) and gamma-valerolactone (50 ml, 528 mmole) were combined and heated under nitrogen for approximately 70 hours at 200-230°. The reaction was cooled and the solid washed with small portions of ethyl alcohol and ether. The solid was then recrystallized from ethyl alcohol/ether/pentane to yield 1-(4-hydroxyphenyl)-5-methyl-pyrrolidin-2-one (7.3 g, m.p. 174°-175°).

Analysis : Calcd. for $C_{11}H_{13}NO_2$ (MW, 191.2):

Theory : C, 69.09% H, 6.85%, N, 7.33%
Found : C, 69.08% H, 6.92%, N, 7.05%

Example 33 : Preparation of 1-(4-acetoxyphenyl)-5-methyl-pyrrolidin-2-one

1-(4-Hydroxyphenyl)-5-methyl-pyrrolidin-2-one (6.0 g, 31.4 mmole) was treated with acetic anhydride (50ml) and pyridine (20ml) and stirred at approximately $100^{0}$ for 2 hours. The product was poured into ice and neutralized with potassium bicarbonate. The mixture was extracted with methylene chloride, dried and evaporated to give a solid which was recrystallized from methylene chlòride/ether/pentane to yield 1-(4-acetoxyphenyl)-5-methyl-pyrrolidin-2-one (3.25 g, m.p. $105\text{-}108^{0}$).

Analysis : Calcd. for $C_{13}H_{15}NO_3$ (MW, 233.3) :

Theory : C, 66.93% H, 6.48%, N, 6.01%
Found : C, 66.93% H, 6.72%, N, 5.79%

Example 34 : Preparation of 1-(4-acetoxy-3-allylphenyl)-pyrrolidin-2-one

1-(4-Hydroxy-3-allylphenyl)-pyrrolidin-2-one (5 g, 28 mmole), triethylamine (7.8 ml, 56mmole), acetic anhydride (5.3 ml, 56 mmole), and tetrahydrofuran (distilled from lithium aluminum hydride, 75 ml) were combined and stirred at room temperature under a nitrogen atmosphere. Additional acetic anhydride (2.6 ml) was added and the reaction stirred overnight.

Saturated aqueous sodium bicarbonate was added and the mixture extracted with methylene chloride several times. The organics were combined, dried over sodium sulfate, and evaporated to give an oil Crystallization from ethyl ether/petroleum ether yielded 1-(4-acetoxy-3-allylphenyl)-pyrrolidin-2-one (2.6 g, m.p. $=48\text{-}50^{0}$).

Example 35 : Preparation of 1-(4-acetoxy-3-(2-oxiranylmethyl)phenyl)-pyrrolidin-2-one

1-(4-Acetoxy-3-allylphenyl)-pyrrolidin-2-one (15 g, 19 mmole) was dissolved into methylene chloride (30 ml), and added to a reaction flask containing m-chloroperoxybenzoic acid (3.3 g, 19 mmole) dissolved in methylene chloride (70 ml). The reaction was maintained at $0^{0}$ under a nitrogen atmosphere and its progress followed by thin layer chromatography.

After approximately 20 hours the reaction was filtered and the filtrate washed with water then aqueous sodium bicarbonate. The organics were then dried over sodium sulfate and evaporated to an oil. The oil was chromatographed over Silica Gel 60 (trademark) eluting with ethyl acetate/light petroleum (40-70%). The appropriate fractions were combined and evaporated to yield an oil. The oil was crystallized from methylene chloride/ethyl ether/light petroleum to yield 1-(4-acetoxy-3-(2-oxiranylmethyl)phenyl)-pyrrolidin-2-one (1.2 g, m.p. 68. 5-70. 5$^{o}$).

Analysis : Calcd. for $C_{15}H_{17}NO_4$ (MW, 275.30)

Theory : C, 65.44% H, 6.23% N, 5.08%
Found : C, 65.18% H, 6.30% N, 5.00%

Example 36 : Preparation of 1-(3-(3-chloro-2-hydroxypropyl)4-hydroxyphenyl)-pyrrolidin-2-one

1-(4-Acetoxy-3-(2-oxiranylmethyl)phenyl)-pyrrolidin-2-one was dissolved into tetrahydrofuran (20ml) and hydrochloric acid (20ml, 6N) added. The reaction was heated to reflux under a nitrogen atmosphere and its progress followed by thin layer chromatography.

The reaction mixture was extracted with methylene chloride and the separated organic layer dried over sodium sulfate. Chromatography over Silica Gel 60 (Trademark) with ethyl acetate/light petroleum (30-50%) and evaporation of the appropriate fractions gave product as a solid. This solid was washed with ethyl ether/light petroleum to yield 1-(3-chloro-2-hydroxypropyl)4-hydroxyphenyl)-pyrrolidin-2-one (0.7 g, m.p. 149.5-151.5$^{o}$).

Analysis : Calcd. for $C_{13}H_{16}NO_3$ (MW.266.73)

Theory : C, 57.98% H, 6.17% N, 5.15%
Found : C, 57.57% H, 6.17% N, 5.14%

Example 37 : Preparation of 2-(2-oxy-1-pyrrolidinyl)phenoxyacetic acid

Example 37(a) : Preparation of 2-(2-(2-oxy-1-pyrrolidinyl)phenoxy)acetonitrile

1-(2-Hydroxyphenyl)-2-pyrrolidinone (30.3 g, 171 mmole) was dissolved under nitrogen in a mixture of tert-butyl alcohol (250 ml sieve dried) and freshly-opened potassium t-butoxide (19.17 g, 171.1 mmole).

Chloroacetonitrile (28.95 g, 380 mmole) was then added and the reaction gently warmed (to prevent solidification) and stirred. After approximately 70 hours the reaction mixture was filtered and the filtrate evaporated leaving an amber oil. This oil was dissolved in ethyl ether/methylene chloride and extracted with dilute base (200 ml of 0.2N NaOH) followed by a water extraction. The organic extracts were dried (over MgS04) and treated with carbon black, filtered through Filtrol (Trademark), and evaporated to an amber oil. The oil was crystallized from methylene chloride/hexane to yield 2-(2-(2-oxo-1-pyrrolidinyl)phenoxy)acetonitrile (19.8 g, m.p. 75.6-78.4$^{o}$.)

Example 37(b) : Preparation of 2-(2-oxy-1-pyrrolidinyl)phenoxyacetic acid

2-(2-(2-Oxo-1-pyrrolidinyl)phenoxy)acetonitrile (4 g, 18 mmole) was heated with hydrochloric acid (50 ml, 6N) at reflux under a nitrogen atmosphere for approximately 20 hours. The solution was allowed to cool and the precipitate filtered, washed with water and dried under vacuum to yield 2-(2-oxo-1-pyrrolidinyl)phenoxyacetic acid (4 g, m.p. 148-149$^{o}$.

Analysis : calcd. for $C_{12}H_{13}NO_4$ (MW 235.23)

Theory : C, 61.27% H, 5.57% N, 5.95%
Found : C, 60.99% H, 5.62% N, 5.87%

Example 38 : Preparation of methyl 1-(1.3-benzodioxol-5-yl)-5-oxo-3-pyrrolidinecarboxylate

1-(1,3-Benzodioxol-5-yl)-5-oxo-3-pyrrolidinecarboxylic acid (10 g, 40 mmole) was dissolved into methanol/chloroform (2:3/200 ml) under a nitrogen atmosphere and trimethyl orthoformate (excess, 15 ml) added as the reaction was maintained at reflux.

An acid catalyst (p-toluenesulfonic acid, approximately 0.5 g) was added and the reaction progress was followed by thin layer chromatography.

The reaction was filtered, diluted with methylene chloride and extracted with aqueous sodium bicarbonate. Evaporation of the organic layer gave crude product which was recrystallized from methanol/methylene chloride to yield methyl 1-(1,3-benzodioxol-5-yl)-5-oxo-3-pyrrolidinecarboxylate (1.8 g, m.p. 146-148$^0$).

Analysis : Calcd. for $C_{13}H_{13}NO_5$ (MW 263.250)

Theory : C, 59.31% H, 4.98% N, 5.32%

Found : C, 59.22% H, 4.99% N, 5.31%

Example 39 : Preparation of 5-(2-oxo-1-pyrrolidinyl)-1,3-benzodioxol-2-carboxylic acid

1-(3,4-Dihydroxphenyl)-pyrrolidin-2-one (9.6 g 50 mmole) and potassium carbonate (35 g) were mixed with tetrahydrofuran (150 ml) under nitrogen. Dichloroacetic acid ethyl ester (6.13 ml, 50 mmole) was added and the mixture stirred for approximately 45 hours. Dry N, N -dimethylformamide (100 ml) was added and the reaction heated and stirred at reflux for approximately 20 hours.

The reaction was then cooled and filtered. The filtrate was evaporated and partitioned between water and methylene chloride. The aqueous phase was separated and acidified (6N HCl) and the precipitate filtered and recrystallized from methyl alcohol/methylene chloride to yield 5-(2-oxo-1-pyrrolidinyl)-1,3-benzodioxol-2-carboxylic acid (1.8 g, m.p. 171.5-175$^0$).

Analysis : Calcd. for $C_{12}H_{11}NO_5$ (MW 267.23)

Theory : C, 57.83% H, 4.45% N, 5.62%
Found : C, 57.74% H, 4.46% N, 5.58%

Example 40 : Preparation of ethyl 5-(2-oxo-1-pyrrolidinyl)-1,3-benzodioxol-2-carboxylate

5-(2-Oxo-1-pyrrolidinyl)-1,3-benzodioxol-2-carboxylic acid (3.5 g, 13 mmole), triethyl orthoformate (13 ml,78 mmole), and p-toluensesulfonic acid (catalyst, 0.05 g) were dissolved under a nitrogen atmosphere in ethyl alcohol (100 ml) and heated to reflux. After 20 hours the mixture was cooled to room temperature and sulfuric acid (3 drops) was added. The mixture was stirred for an additional three hours then aqueous sodium bicarbonate was added. It was then extracted several times with methylene chloride and the organic layer dried (over sodium sulfate) and evaporated to crude product. Chromatography over Silica Gel 60 (Trademark) eluting with ethyl acetate/light petroleum (30-40%) and evaporation of the appropriate fractions gave a solid. Recrystalization from ethyl ether/light petroleum yielded ethyl 5-(2-oxo-1-pyrrolidinyl)-1,3-benxzodioxol-2-carboxylate (0.7 g, m.p. 49-50$^{O}$).

Analysis : Calcd. for $C_{14}H_{15}NO_5$ (MW, 277.27)

Theory : C, 60.64% H, 5.45% N, 5.05%
Found : C, 60.66% H, 5.48% N, 5.01%

Example 41 : Preparation of ethyl 1-(1,3-benzodioxol-5-yl)-5-oxo-3-pyrrolidine carboxylate.

1-(1,3-Benzodioxol-5-yl)-5-oxo-3-pyrrolidinecarboxylic acid (4 g, 16 mmole) was dissolved into ethanol (200 ml) and a catalytic amount of sulfuric acid added (5 drops). The reaction was stirred at room temperature under a nitrogen atmosphere for four days.

The reaction was then diluted with methylene choloride (50 ml) and decolourized with carbon black, filtered through Celite (trademark) and evaporated to a dark oil which crystallized.

The product was purified by chromatography over Silica Gel 60 (trademark) eluting with ethyl acetate/light petroleum. The fractions containing product (as determined by thin layer chromatography) were combined and concentrated giving an oil which crystallized to yield ethyl 1-(1,3-benzodioxol-5-yl)-5-oxo-3-pyrrolidinecarboxylate (2.5 g, m.p. = 65-68$^{O}$).

Analysis : Calcd. for $C_{14}H_{15}NO_5$ (MW 277.277)

Theory : C, 60.64% H, 5.45% N, 5.05%.
Found : C, 60.48% H, 5.47% N, 4.99%.

Example 42 : Preparation of 1-(1,3-benzodioxol-5-yl)-4-hydroxymethyl-pyrrolidin-2-one

Methyl 1-(1,3-benzodioxol-5-yl)-5-oxo-3-pyrrolidinecarboxylate (4.25 g, 16.1 mmole) was mixed with tetrahydrofuran (distilled from lithium aluminum hydride, 180ml) and transferred to a reaction flask containing lithium borohydride (0.26g, 11.9 mmole). The reaction was maintained under a nitrogen atmosphere and heated to reflux. Toluene (5ml) was added and the tetrahydrofuran removed by reflux over four hours. The reaction was allowed to cool overnight and the toluene poured off the residue which was then hydrolised using aqueous hydrochloric acid (12ml, 1N).

Solids were filtered and washed with dilute acid (2x30ml 0.1 N HCl), saturated aqueous sodium bicarbonate (3x30ml) and water (30ml). The product was dried to yield 1-(1,3-benzodioxol-5-yl)-4-hydroxymethyl-pyrrolidin-2-one (1.9g, m.p. 114-117.5$^{o}$).

Analysis : Calcd. for $C_{12}H_{13}NO_4$ (MW 235.240).

Theory : C, 61.27% H, 5.57% N, 5.95%
Found : C, 61.28% H, 5.59% N, 5.92%

Example 43: Analgesic Activity

A. Acetic Acid Writhing Test (AAWT)

Using the procedure described by Koster et al. in Fed. Proc. 18, 412 (1959) and Vinegar et al. in Handbook of Experimental Pharmacology, 50-2, ch. 26, Anti-inflammatory Drugs, Ed JR Vane and SH Ferreria (1978), the acetic acid writhing test was performed, using both the mouse and the rat, to demonstrate the mild analgesic activity of the compounds of formula (I).

B. Modified Trysin Hyperalgesic Assay (THA)

This assay quantitatively measures analgesia and is designed to be unaffected by compounds possessing anti-inflammatory activity. The procedure described by Vinegar et al. in Eur. J] Pharmacol. 37, 23 (1976) was used to demonstrate the analgesic activity of the compounds of formula (I) and of certain known analgesics. The analgesic agents were administered 30 minutes after the administration of trypsin. In addition, a modification of Vinegar's published assay was carried out, comprising the administration of analgesic agent preceding subplantar injectin of trypsin (0.10 ml) of 10% solution of trypsin in pyrogen-free water by 15 minutes. In both THA's, pain scores were determined 60 minutes after trypsin injection. The results of the modification was to increase the sensitivity of the THA to the mild analgesic action of the agents. Results are given in TABLE 1.

Table 1

| Compound | $ED_{50}$ mg/kg |
|---|---|
| Example 1 | 80 |
| " 4 | 46 |
| " 5 | 60 |
| " 7 | 45 |
| " 27 | 40 |

C. Rat Hot Plate Assay

The rat hot plate assay incorporated 2 modifications of the mouse hot plate assay originally described by Eddy et al, J. Pharmacol. Exp. Ther. 98, 121-137 (1950). The first modification was enlargement of the diameter of the cylindrical (water filled) copper plate to 25.0 cm to accommodate rats instead of mice.

The second modification was the use of a temperature controller to regulate a 250 watt infrared heat lamp which was activated via a thermistor probe attached to the undersurface of the top of the copper plate. The surface temperature was thus maintained at 45 $\pm$ 1.0$^{o}$C (N=28 measurements of the hot plate temperature under experimental conditions). The time in which a rat placed on the hotplate responded by lifting, shaking or licking either or its hind or forelimbs was recorded in tenths of a second.

Only animals responding in pretest within 6-13 seconds were used in the studies. Drugs were suspended in 0.5% sodium carboxymethylcellulose and administered orally, by gavage, in a volume of 1.00 ml/100 g.b wt. 60 min prior to testing. Animals responded in less than 18.3 seconds were considered unprotected and those which did not respond within 18.3 seconds were considered protected. The reaction time of 18.3 seconds represented the sum of the mean protest times of 40 untreated rats plus the time of 3 standard deviations of the mean.$ED_{50}$'s and their standard errors were estimated from a graph of the dose-response curves using the method of Miller and Tainter (Proc.Soc.Exp.Biol.Med. 57, 261-262 (1944). Following this procedure the analgesic activity of the compounds of formula (I) was compared to that of standard analgesic drugs.

Results obtained are given in TABLE 2.

Table 2

| Compound | $ED_{50}$ mg/kg |
|---|---|
| Example 1 | 120 |
| " 5 | 120 |
| " 7 | 30 |
| " 28 | 120 |

Example 44: Acute Anti-inflammatory Activity:Carrageenin Pleurisy Assay. (CPA)

Following the procedure described by Vinegar et al. in Proc.Soc.Exp.Biol.Med. 151, 556, (1976), the actue anti-inflammatory activityof compounds of formula (I) was compared with that of known anti-inflammatory drugs in the rat. The average 3 hour exudate volume for each drug treated group was determined and the % inhibition relative to solvent-fed control animals calculated, the $ED_{50}$ being the dose required to reduce the 3 hour exudate volume by 50%. Results obtained are given in TABLE 3.

Table 3

| Compound | $ED_{50}$ mg/kg |
|---|---|
| Example 1 | 96 |
| " 2 | 57 |
| " 4 | 75 |
| " 5 | 42 |
| " 6 | 80 |
| " 7 | 34 |
| " 10 | 95 |
| " 18 | 19 |
| " 20 | 78 |
| " 21 | 41 |
| " 24 | 29 |
| " 26 | 39 |
| " 27 | 35 |
| " 28 | 62 |
| " 31 | 39 |
| " 37 | 50 |

Example 45: Antipyretic Activity

The Yeast-Induced Hyperthermia Assay was used according to the procedure described by Khalili-Varasteh et al. in Arch.Int.Pharmacodyn. 219, 149-159, (1976) to demonstrate the antipyretic activity of compounds of formula (I) and certain known antipyretics in the rat.

Example 46: Pharmaceutical Formulations

A. Capsule

| Ingredient | Amount per capsule (mg |
|---|---|
| Compound of formula (I) | 325.0 |
| Lactose | 174.0 |
| Corn Starch | 174.0 |
| Stearic Acid | 2.0 |

The finely ground active compound is mixed with the powdered excipients lactose, corn starch and stearic acid and packed into a gelatin capsule.

B. Tablet

| Ingredient | Amount per tablet (mg) |
|---|---|
| Compound of formula (I) | 325.0 |
| Lactose | 125.0 |
| Corn Starch | 50.0 |
| Polyvinylpyrrolidone | 3.0 |
| Stearic acid | 1.0 |
| Magnesium stearate | 1.0 |

The active compound is finely ground and intimately mixed with the powdered excipients lactose, corn strach, polyvinylpyrrolidone, magnesium stearate and stearic acid. The formulation is then compressed to afford one tablet weighing 505 mg.

C. Suppository

| Ingredient | Amount per suppository |
|---|---|
| Compound of formula (I) | 325.0 mg |
| Cocoa Butter, q.s. | 2.0 g |
| or Wecobee Base | |

Wecobee is the trade name of a hydrogenated carboxylic acid.

Example 47 : Toxicity:

The compounds of formula (I) were found to be non-toxic when administered in dosag the therapeutic range intended for use.

1. A pharmaceutical formulation comprising a compound of formula (I) or an acid addition salt thereof in association with a pharmaceutically acceptable carrier therefor -

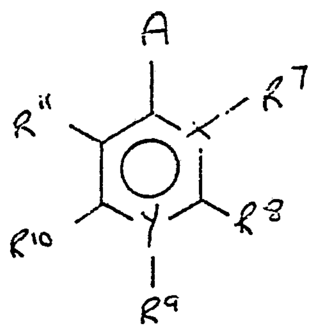

(I)

wherein, A is $NHCH_2CO_2R^5$, or 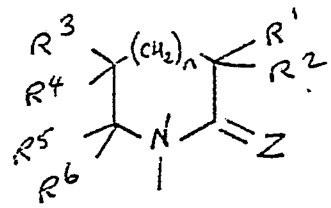

and

$R^1$ and $R^2$ are the same or different and each may be hydrogen, alkyl, alkenyl, alkoxy, $CO_2$-alkyl, hydroxy and $CO_2H$; $R^3$ and $R^4$ are the same or different and each may be hydrogen, alkyl, alkenyl, aralkyl, hydroxy, alkoxy, $CO_2H$, $CO_2$ alkyl or halogen;

$R^5$ and $R^6$ are the same or different and each may be hydrogen or alkyl;

$R^7$ is hydrogen, alkyl, hydroxy, alkoxy, O-alkenyl, O-alkynyl, alkyl substituted by halogen, amino, nitro, $OCH_2CO_2H$ or halogen;

$R^8$ is hydrogen, hydroxy, alkyl, alkenyl, aralkyl, O-alkyl, alkyl substituted by halogen, halogen, O-acyl, nitro, amino, $CH\text{-}\overset{O}{CH}-CH_2$, $CH_2CH(OH)CH_2Cl$, $CO_2H$, or 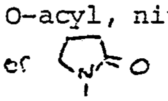 ;

$R^9$ is hydrogen, alkyl, O-alkyl, O-alkenyl, hydroxy, amino, cyano, acylamino, O-acyl or $CO_2H$;

$R^8$ and $R^9$ together may be, $-CH=N-NH-$, $-(CH_2)_3$, $-O(CH_2)_2O-$, $-OCH(CH_3)O-$, $-OCF_2O-$ $-OC(CH_3)_2O-$, $-OCH_2O$, $-OCH(CO_2Et)O-$, $-OCH(CO_2H)O$, or $-OC(CH_3)(CH_3)-O-$;

$R^{10}$ is hydrogen, alkyl, alkyl substituted by halogen, O-alkyl, or halogen;

$R^{11}$ is hydrogen;

n is O or 1;

X is a nitrogen or a carbon atom, and Y is a nitrogen or carbon atom, provided that when one of X and Y is nitrogen the other is carbon; and

Z is oxygen or NH;

provided that when $R^5$, $R^6$, $R^7$, $R^{10}$ and $R^{11}$ are all hydrogen and $R^8$ and $R^9$ together are $-OCH_2O-$, $R^1$ to $R^4$ are not all hydrogen.

2. A pharmaceutical formulation comprising a compound of formula (II) or an acid addition salt thereof in association with a pharmaceutically acceptable carrier therefor -

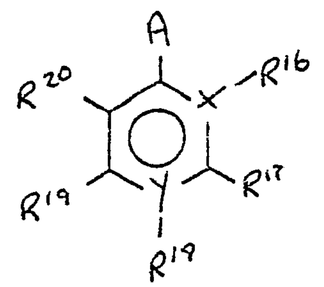

(II)

wherein, A is $NH(CH_2)_3CO_2H$ or

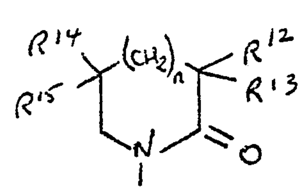

and $R^{12}$ and $R^{13}$ are the same or different and each is hydrogen or alkyl; $R^{14}$ and $R^{15}$ are the same or different and each is hydrogen, halogen, or alkyl; $R^{16}$ is hydrogen, hydroxy, alkyloxy, O-alkenyl or O-alkynyl;

$R^{17}$ is hydrogen, hydroxy, alkyl, O-alkyl, alkyl substituted with halogen, halogen, or alkenyl; $R^{18}$ is hydrogen, alkyl, O-alkyl, or O-alkenyl; $R^{19}$ is hydrogen, alkyl or O-alkyl; $R^{20}$ is hydrogen; $R^{17}$ and $R^{18}$ together may be $-CH=N-NH-$, $-(CH_2)_3-$, $-O-(CH_2)_2O$, $-O\ CH(CH_3)-O-$ $OCF_2O-$, $-OC(CH_3)_2O-$, $-OCH_2O-$; n is 0 or 1; X is a nitrogen or carbon atom, and Y is a nitrogen or carbon atom, provided that, when one of X and Y is nitrogen, then the other is carbon;

with the proviso that when $R^{16}$, $R^{19}$ and $R^{20}$ are all hydrogen and $R^{17}$ and $R^{18}$ together are $-OCH_2O-$, $R^{12}$ to $R^{15}$ are not all hydrogen.

3. A pharmaceutical formulation comprising a compound of formula (III) or an acid addition salt thereof in association with a pharmaceutically acceptable carrier therefor.

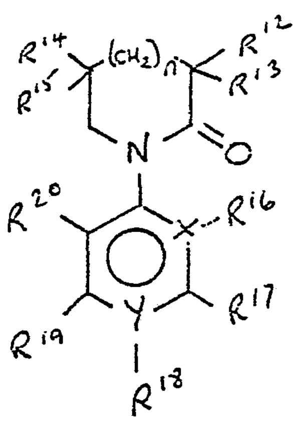

(III)

wherein, $R^{12}$, $R^{13}$, $R^{14}$, $R^{15}$, $R^{16}$, $R^{17}$, $R^{18}$, $R^{19}$, $R^{20}$, X, Y and n, are as hereinbefore defined, in association with a pharmaceutically acceptable carrier thereof and with the proviso that when $R^{16}$, $R^{19}$ and $R^{20}$ are all hydrogen, and $R^{17}$ and $R^{18}$ together are $-OCH_2O-$, $R^{12}$ to $R^{15}$ are not all hydrogen.

4. A pharmaceutical formulation as claimed in claim 3 wherein the compound of formula (III) is selected from the group consisting of -

1-(1,3-benzodioxol-5-yl)-4-methyl-pyrrolidin-2-one
1-(4-allyloxyphenyl)-pyrrolidin-2-one
trans-1-(1,3-benzodioxol-5-yl)-3,4-dimethyl-pyrrolidin-2-one
1-(2,5-dimethoxyphenyl)-pyrrolidin-2-one
1-(1H-indazol-5-yl)-pyrrolidin-2-one
1-(5-indanyl)-pyrollidin-2-one
1-(1,3-benzodioxol-5-yl)-2-piperidin-2-one
1-(3,5-dimethoxyphenyl)-pyrrolidin-2-one
1-(2,2-dimethyl-1,3-benzodioxol-5-yl)-pyrrolidin-2-one
1-(2,2-difluoro-1,3-benzodioxol-5-yl)-pyrrolidin-2-one
1-(2-methyl-1,3-benzodioxol-5-yl)-pyrrolidin-2-one
1-(4-hydroxy-3-methoxyphenyl)-pyrrolidin-2-one
1-(3-hydroxy-4-methoxyphenyl)-pyrrolidin -2-one
1-(2-methyl-4-pyridyl)-pyrrolidin-2-one
1-(4-pyridyl)-piperidin-2-one
1-(3,4-diethoxyphenyl)-pyrrolidin-2-one
1-(4-pyridyl)-pyrrolidin-2-one
1-(3-ethylphenyl)-pyrrolidin-2-one
1-(2-pyridyl)-pyrrolidin-2-one
3-methyl-1-phenyl-pyrrolidin-2-one
1-(3,5-dimethyl-2-pyridyl)-pyrrolidin-2-one
1-(3-methyl-2-pyridyl)-pyrrolidin-2-one
1-(2,3-dihydro-1,4-benzodioxin-6-yl)-pyrrolidin-2-one
1-(1,3-benxodioxol-5-yl)-3-methyl-pyrrolidin-2-one

1-(-2-allyloxyphenyl)-pyrrolidin-2-one
1-(2-hydroxy-3-allylphenyl)-pyrrolidin-2-one
1-(2-prop-2-yn-yloxyphenyl)-pyrrolidin-2-one
1-(2-but-2-yn-yloxylphenyl)-pyrrolidin-2-one
1-(2-(1-methyl-prop-2-yn-yl)oxyphenyl)-pyrrolidin-2-one
1-(4-methylphenyl)-pyrrolidin-2-one
1-(4-fluorophenyl)-pyrrolidin-2-one
1-(4-trifluoromethylphenyl)-pyrrolidin-2-one
1-(3-methylphenyl)-pyrrolidin-2-one
1-phenyl-piperidin-2-one
1-(3-methoxyphenyl)-pyrrolidin-2-one
1-(3-chlorophenyl)-pyrrolidin-2-one
1-(3,4-dimethoxyphenyl)pyrrolidin-2-one

5. A pharmaceutical formulation as claimed in claim 1 in unit dosage form.

6. A pharmaceutical formulation as claimed in claim 1 in the form of a tablet, syrup, suppository or sterile aqueous preparation.

7. A pharmaceutical formulation as claimed in claim 1, wherein the compound of formula (I) is combined with a therapuetic ingredient.

8. A compound of formula (IA)

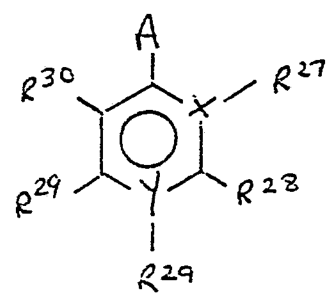

(IA)

wherein A is $NH(CH_2)_3\ CO_2R^{25}$ or 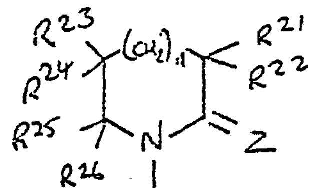

$R^{21}$, $R^{22}$, $R^{23}$ and $R^{24}$ are the same or different and each may be hydrogen, alkyl, alkenyl, alkoxy or $CO_2$ alkyl.

$R^{25}$ and $R^{26}$ are the same or different and each may be hydrogen or alkyl.

$R^{27}$ is hydrogen, alkyl, hydroxy, alkoxy, O-alkenyl, or O-alkynyl.

$R^{28}$ is hydrogen, hydroxy, alkenyl, alkoxy, alkyl, O-acyl, CH-CH $CH_2$, $CH_2CH(OH)CH_2Cl$ or 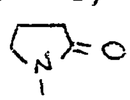

$R^{29}$ is hydrogen, O-alkenyl, hydroxy, O-acyl or alkoxy; or

$R^{28}$ and $R^{29}$ together may be -CH=N-NH-, $-(CH_2)_3-$, $-O(CH_2)_2O-$,

$-OCF_2O-$, $-OC(CH_3)_2O-$, $-OCH_2O$;

$R^{30}$ is hydrogen, alkyl, or O-alkyl;
$R^{31}$ is hydrogen;
n is 0 or 1,

X is a nitrogen or a carbon atom, and Y is a nitrogen or carbon atom, provided that when one of X and Y is nitrogen the other is carbon;

Z is oxygen or NH,
with the proviso that when $R^{25}$, $R^{26}$, $R^{27}$, $R^{30}$, and $R^{31}$ are all hydrogen and $R^{28}$ and $R^{29}$ together are $OCH_2O$, $R^{21}$-$R^{24}$ are not all hydrogen.

9. A compound of formula (IA) as claimed in claim 8, wherein A is

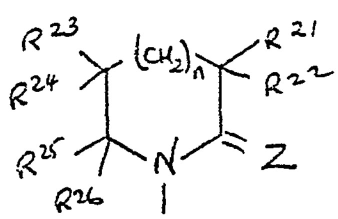

and $R^{21}$ to $R^{31}$, n, x, Y and Z are as hereinbefore defined.

10. A compound of formula (IA) as claimed in claim 8, wherein the compound of formula (IA) is selected from the group consisting of

1-(1,3-benzodioxol-5-yl)-4-methyl-pyrrolidin-2-one
1-(4-allyloxyphenyl)-pyrrolidin-2-one
1-(1,3-benzodioxol-5-yl)-3,3-dimethyl-pyrrolidin-2-one
<u>trans</u>-1-(1,3-benzodioxol-5-yl)-3,4-dimethyl-pyrrolidin-2-one
1-(2,5-dimethoxyphenyl)-pyrrolidin-2-one
1-(1H-indazol-5-yl)-pyrrolidin-2-one
1-(5-indanyl)-pyrrolidin-2-one
1-(1,3-benzodioxol-5-yl)-piperidin-2-one
1-(3,5-dimethoxyphenyl)-pyrrolidin-2-one
1-(2,2-dimethyl-1,3-benzodioxol-5-yl)-pyrrolidin-2-one
1-(2,2-difluoro-1,3-benzodioxol-5-yl)-pyrrolidin-2-one
1-(2-methyl-1,3-benzodioxol-5-yl)-pyrrolidin-2-one
1-(4-hydroxy-3-methoxyphenyl)-pyrrolidin-2-one
1-(3-hydroxy-4-methoxyphenyl)-pyrolidin-2-one
1-(2-methyl-4-pyridyl)-pyrrolidin-2-one
1-(4-pyridyl)-piperidin-2-one
1-(3,4-diethoxyphenyl)-pyrrolidin-2-one
1-(4-pyridyl)-pyrrolidin-2-one
1-(3-ethylphenyl)-pyrrolidin-2-one
1-(2-pyridyl)-pyrrolidin-2-one
3-methyl-1-phenyl-pyrrolidin-2-one
1-(3,5-dimethyl-2-pyridyl)-pyrrolidin-2-one
1-(3-methyl-2-pyridyl)-pyrrolidin-2-one
1-(2,3-dihydro-1,4-benzodioxin-6-yl)-pyrrolidin-2-one
1-(1,3-benzodioxol-5-yl)-3-methyl-pyrrolidin-2-one
1-(2-allyloxyphenyl)-pyrrolidin-2-one
1-(2-hydroxy-3-allylphenyl)-pyrrolidin-2-one
1-(2-prop-1-yn-yloxphenyl)-pyrrolidin-2-one
1-(2-but-2-yn-yloxylphenyl)-pyrrolidin-2-one
1-(2-(1-methyl-prop-2-yn-yl)oxyphenyl)-pyrrolidin-2-one
4-(1,3-benzodioxol-5-yl)amino butyric acid.

3-allyl-1-(1,3-benzodioxol-5-yl)-pyrrolidin-2-one
1-(1,3-benzodioxol-5-yl)-3-ethyl-pyrrolidin-2-one
2-allyl-4-(2-oxo-1-pyrrolidinyl)-phenyl acetate
1-(4-acetoxyphenyl)-5-methyl-pyrrolidin-2-one
1-(4-hydroxyphenyl)-5-methyl-pyrrolidin-2-one
1-(4-acetoxy-3-allyl phenyl)-pyrrolidin-2-one
1-(4-acetoxy-3-(2-oxiranyl methyl)-phenyl)-pyrrolidin-2-one
1-[3-(3-chloro-2-hydroxylpropyl)-4-hydroxyphenyl]-pyrrolidin-2-one

2-(2-oxy-1-pyrrolidinyl)-phenoxyacetic acid
Methyl 1-(1,3-benzodioxol-5-yl)-5-oxo-3-pyrrolidine carboxylate
5-(2-oxo-1-pyrrolidinyl-1,3-benzodioxol-2-carboxylic acid
Ethyl 5-(2-oxo-1-pyrrolidinyl)-1,3-benzodioxol-2-carboxylate
Ethyl 1-(1,3-benzodioxol-5-yl)-5-oxo-3-pyrrolidine carboxylate
1-(3,4-diethoxyphenyl)-pyrrolidin-2-one
1-(4-propionyloxyphenyl)-pyrrolidin-2-one
1-(4-acetoxy-3-(2-oxiranyl methyl)-phenyl)-pyrrolidin-2-one
1-(3-(3-chloro-2-hydroxypropyl)-4-hydroxyphenyl)-pyrrolidin-2-one

11. A process for the preparation of a compound of formula (I) wherein,

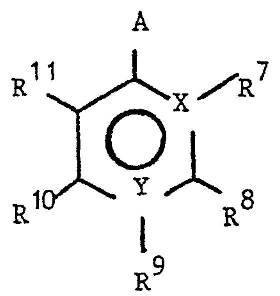

(I)

wherein A is 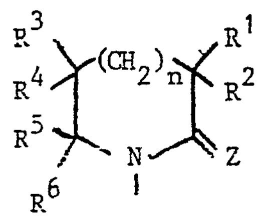 , and

$R^1$ and $R^2$ are the same or different and each may be hydrogen, alkyl, alkenyl, alkoxy, $CO_2$-alkyl, hydroxy and $CO_2H$, $R^3$ and $R^4$ are the same or different and each may be hydrogen, alkyl, alkenyl, aralkyl, hydroxy, alkoxy, $CO_2H$, $CO_2$ alkyl or halogen, $R^5$ and $R^6$ are the same or different and each may be hydrogen or alkyl;

$R^7$ is hydrogen, alkyl, hydroxy, alkoxy, O-alkenyl, O-alkynyl, alkyl substituted by halogen, amino, nitro, $OCH_2$ $CO_2H$ or halogen;

$R^8$ is hydrogen, hydroxy, alkyl, alkenyl, aralkyl, O-alkyl, alkyl substituted by halogen, halogen, O-acyl, nitro, amino,

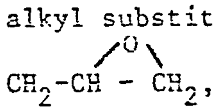, $CH_2$ CH (OH) $CH_2$ Cl or 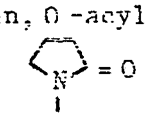

$R^9$ is hydrogen, alkyl, O-alkyl, O-alkenyl, hydroxy, amino, cyano, acylamino, O-acyl or $CO_2H$;

$R^8$ and $R^9$ together may be, -CH=N-NH-, $-(CH_2)_3$, $-O(CH_2)_2O-$, $-OCH(CH_3)O$, $-OCF_2O-$, $-OC(CH_3)_2O-$, $-OCH_2O$, $-OCH(CO_2Et)O-$, $-OCH(CO_2H)O-$, or 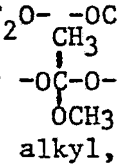;

$R^{10}$ is hydrogen, alkyl, alkyl substituted by halogen, O-alkyl, or halogen;

$R^{11}$ is hydrogen;

n is 0 or 1;

X is a nitrogen or a carbon atom, and Y is a nitrogen or carbon atom, provided that when one of X and Y is nitrogen the other is carbon; and

Z is oxygen

provided that when $R^5$, $R^6$, $R^7$, $R^{10}$ and $R^{11}$ are all hydrogen and $R^8$ and $R^9$ together are $-OCH_2O-$, $R^1$ to $R^4$ are not all hydrogen, which method comprises, cyclisation of a compound of formula (IV) or a compound of formula (V),

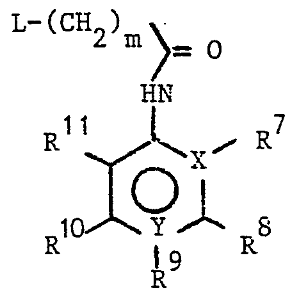

(IV)

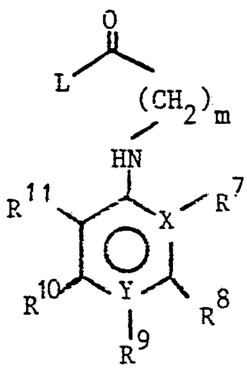

(V)

wherein L is a standard leaving group, and m is 3 or 4.

12. A process as claimed in claim 11, characterised in that one cyclises a compound of formula (IV) or formula (V) in the presence of an aqueous alkali, metal hydride, hydroxide or alkoxide and a phase transfer catalyst.

13. A process as claimed in claim 12, characterised in that one cyclises a compound of formula (IV) or formula (V) in the presence of aqueous sodium hydroxide and triethylbenzyl ammonium chloride.

14. A process for the preparation of a compound of formula (III),

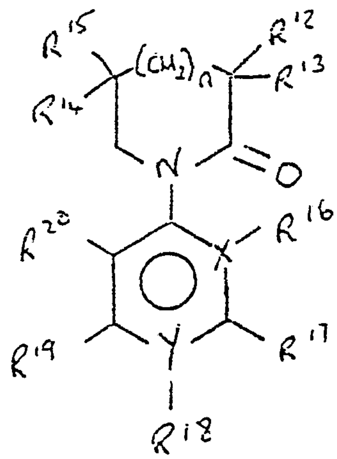

(III)

wherein $R^{12}$ and $R^{13}$ are the same or different and each is hydrogen or alkyl; $R^{14}$ and $R^{15}$ are the same or different and each is hydrogen, halogen, or alkyl; $R^{16}$ is hydrogen, hydroxy, alkyloxy, O-alkenyl or O-alkynyl; $R^{17}$ is hydrogen, hydroxy, alkyl, O-alkyl, alkyl substitute with halogen, halogen, or alkenyl; $R^{18}$ is hydrogen, alkyl, O-alkyl, or O-alkenyl; $R^{19}$ is hydrogen, alkyl or O-alkyl; $R^{20}$ is hydrogen; $R^{17}$ and $R^{18}$ together may be -CH=N-NH-, $-(CH_2)_3-$, $-O-(CH_2)_2O$, $-O\,CH(CH_3)-O-$, $OCF_2O-$, $-OC(CH_3)_2O-$, $-OCH_2O-$, n is 0 or 1; X is a nitrogen or carbon atom, and Y is a nitrogen or carbon atom, provided that, when one of X and Y is nitrogen, then the other is carbon; with the proviso that when $R^{16}$, $R^{19}$ and $R^{20}$ are all hydrogen and $R^{17}$ and $R^{18}$ together are $-OCH_2O-$, $R^{12}$ to $R^{15}$ are not all hydrogen, which method comprises -

(a) where, in formula (III), n is O and one or more of $R^{12}$ - $R^{13}$ is an alkyl group, reaction of a compound of formula (VI) wherein $R^{16}$ to $R^{20}$, X and Y are as hereinbefore defined with a base and an alkyl donor;

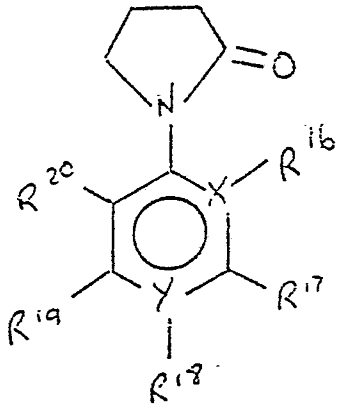

(VI)

(b) where, in formula (III) n is O and one of $R^{16}$ and $R^{18}$ is O-alkenyl or O-alkynyl, reaction of a corresponding compound of formula (VII) or formula (VIII) with an alkenyl or alkynyl donor,

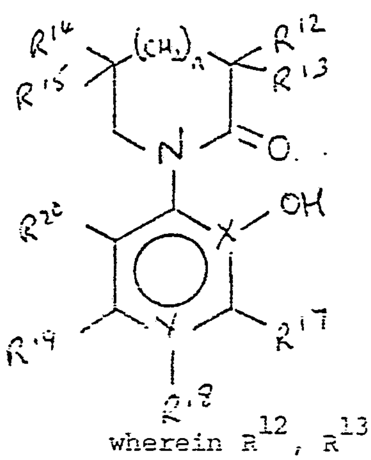

(VII)

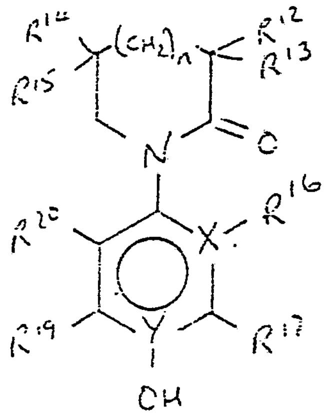

(VIII)

wherein $R^{12}$, $R^{13}$, $R^{14}$, $R^{15}$, $R^{17}$, $R^{18}$, $R^{19}$, $R^{2o}$, n, X and Y are as hereinbefore defined, provided that in (VII) $R^{18}$ is hydrogen and X is carbon, and in (VIII) R 16 is hydrogen and Y is carbon;

(c) where, in formula (III), n is O, a displacement reaction between a compound of formula (X) and pyrrolidinone anion (XI).

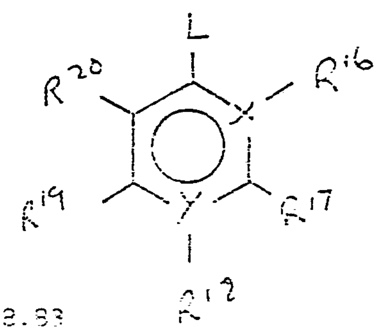

(X)

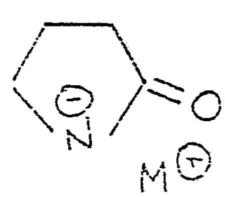

(XI)

wherein L is a standard leaving group, $M^{(+)}$ is an alkali metal or alkaline earth metal cation and $R^{16}$ to $R^{20}$, X and Y are as hereinbefore defined;

(d) where, in formula (III) $R^{12}$ to $R^{15}$ are all hydrogen, reaction of a compound of formula (XII) with γ-butyrolactone

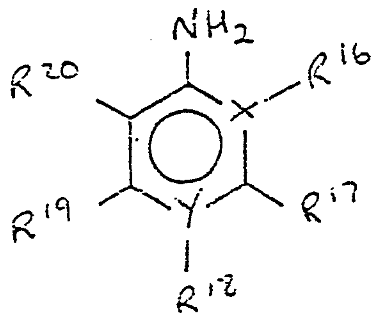

(XII)

wherein X, Y and $R^{16}$ to $R^{20}$ are as hereinbefore defined.

(e) where in formula (III), one of $R^{12}$ and $R^{13}$ is methyl and the other is hydrogen and $R^{14}$ and $R^{15}$ are both hydrogen, reaction of a compound of formula (XII) with 3-methyl-γ-butyrolactone.

(f) where in formula (III) $R^{12}$, $R^{13}$, $R^{14}$, $R^{15}$, $R^{18}$, $R^{19}$ and $R^{20}$ are all hydrogen, $R^{16}$ is hydroxy, $R^{17}$ is alkyl, n is 0 and X and Y are both carbon, Claisen rearrangement of a compound of formula (XV)

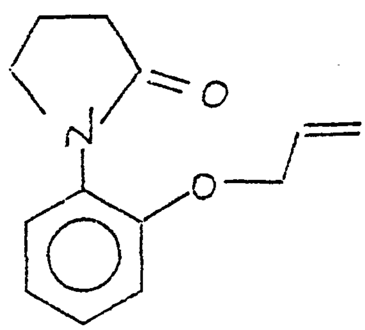

(XV)

(g) where, in formula (III) $R^{14}$ or $R^{15}$ is alkyl, reaction of a compound of formula (XVI), wherein P is alkyl and X, Y and $R^{16}$ to $R^{20}$ are as hereinbefore defined, with a suitable dialkyl sulphoxonium methylide

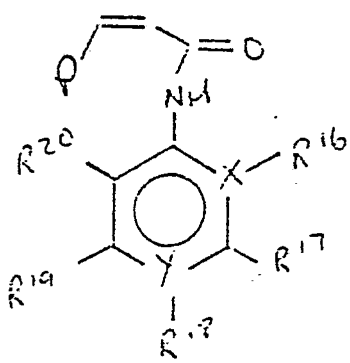

(XVI)

(h) reduction of a compound of formula (XVII), wherein X, Y, n and $R^{12}$ to $R^{20}$ are all as hereinbefore defined.

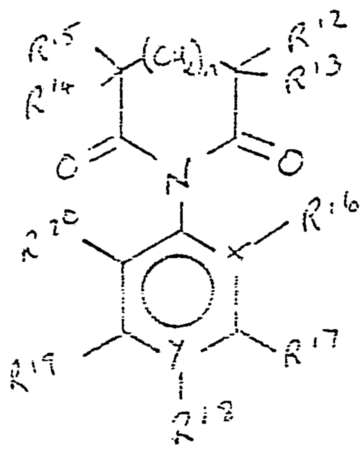

(XVII)

i) where, in formula (iii) $R^{12}$, $R^{13}$, $R^{14}$, $R^{15}$ are all hydrogen cyclisation of a compound of formula (XIII)

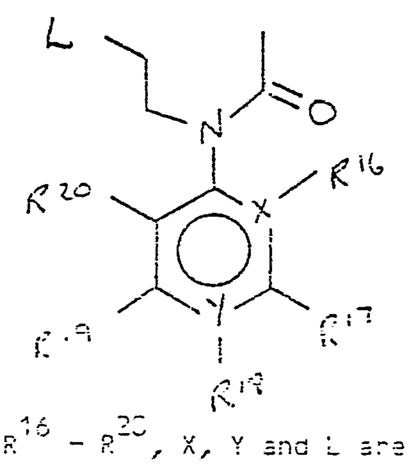

(XIII)

wherein $R^{16}$ - $R^{20}$, X, Y and L are as hereinbefore defined.

BAD ORIGINAL

15. A process for the preparation of a compound of formula (I) wherein A is $NH(CH_2)_3CO_2H$ which comprises base catalysed ring opening of a compound of formula (VI)

16. A process for the preparation of a compound of formula (I) wherein A is $NH(CH_2)_3CO_2$-alkyl which comprises reaction of a compound of formula (XII) with a suitable reagent.

17. A process as claimed in claim 16 wherein the suitable reagent is $Br(CH_2)_3CO_2Et$.

18. A process for the preparation of a compound of formula (I) wherein A is $NH(CH_2)_3CO_2H$ which comprises hydrolysis of a compound of formula (I) wherein A is $NH(CH_2)_3CO_2R^5$ and $R^5$ isalkyl.

19. A process for the preparation of a formulation of a compound of formula (I) which comprises admixture of a compound of formula (I) with a pharmaceutically acceptable carrier thereof.

20. A process for the preparation of an analgesic, anti-inflammatory or anti-pyretic agent, which comprises reacting a compound of of formula (IV) or a compound of formula (V) and incorporating the resulting compound of formula (I) in a pharmaceutically acceptable carrier therefor.

21. A process as defined in any of claims 11 to 14, when used to prepare a compound selected from the group consisting of,

1.(1,3-benzodioxol-5-yl)-4-methyl-pyrrolidin-2-one

1-(4-allyloxyphenyl)-pyrrolidin-2-one

1-(1,3-benzodioxol-5-yl)-3,3-dimethyl-pyrrolidin-2-one

trans-1-(1,3-benzodioxol-5-yl)-3,4-dimethyl-pyrrolidin-2-one

1-(2,5-dimethoxyphenyl)-pyrrolidin-2-one

1-(1H-indazol-5-yl)-pyrrolidin-2-one

1-(5-indanyl)-pyrrolidin-2-one

1-(1,3-benzodioxol-5-yl)-piperidin-2-one

1-(3,5-dimethoxyphenyl)-pyrrolidin-2-one

1-(2,2-dimethyl-1,3-benzodioxol-5-yl)-pyrrolidin-2-one

1-(2,2-difluoro-1,3-benzodioxol-5-yl)-pyrrolidin-2-one

1-(2-methyl-1,3-benzodioxol-5-yl)-pyrrolidin-2-one

1-(4-hydroxy-3-methoxyphenyl)-pyrrolidin-2-one

1-(3-hydroxy-4-methoxyphenyl)-pyrolidin-2-one

1-(2-methyl-4-pyridyl)-pyrrolidin-2-one

1-(4-pyridyl)-piperidin-2-one

1-(3,4-diethoxyphenyl)-pyrrolidin-2-one

1-(4-pyridyl)-pyrrolidin-2-one

1-(3-ethylphenyl)-pyrrolidin-2-one

1-(2-pyridyl)-pyrrolidin-2-one

3-methyl-1-phenyl-pyrrolidin-2-one

1-(3,5-dimethyl-2-pyridyl)pyrrolidin-2-one

1-(3-methyl-2-pyridyl)pyrrolidin-2-one

1-(2,3-dihydro-1,4-benzodioxin-6-yl)-pyrrolidin-2-one

1-(1,3-benzodioxol-5-yl)-3-methyl-pyrrolidin-2-one

1-(2-allyloxyphenyl)-pyrrolidin-2-one

1-(2-hydroxy-3-allylphenyl)-pyrrolidin-2-one

1-(2-prop-1-yn-yloxyphenyl)-pyrrolidin-2-one

1-(2-but-2-yn-yloxylphenyl)-pyrrolidin-2-one

1-(2-(1-methyl-prop-2-yn-yl)oxyphenyl)-pyrrolidin-2-one

3-allyl-1-(1,3-benzodioxol-5-yl)-pyrrolidin-2-one

1-(1,3-benzodioxol-5-yl)-3-ethyl-pyrrolidin-2-one

2-allyl-4-(2-oxo-1-pyrrolidinyl)-phenyl acetate

1-(4-acetoxyphenyl)-5-methyl-pyrrolidin-2-one

1-(4-hydroxyphenyl)-5-methyl-pyrrolidin-2-one

1-(4-acetoxy-3-allyl phenyl)-pyrrolidin-2-one

1-(4-acetoxy-3-(2-oxiranyl methyl)phenyl)-pyrrolidin-2-one

1-[3-(3-chloro-2-hydroxylpropyl)-4-hydroxyphenyl]-pyrrolidin-2-one

2-(2-oxy-1-pyrrolidinyl)phenoxyacetic acid

Methyl 1-(1,3-benzodioxol-5-yl)-5-oxo-3-pyrrolidine carboxylate

5-(2-oxo-1-pyrrolidinyl)-1,3-benzodioxol-2-carboxylic acid

Ethyl 5-(2-oxo-1-pyrrolidinyl)-1,3-benzodioxol-2-carboxylate

Ethyl 1-(1,3-benzodioxol-5-yl)-5-oxo-3-pyrrolidine carboxylate

1-(3,4-diethoxyphenyl)-pyrrolidin-2-one

1-(4-propionylaxyphenyl)-pyrrolidin-2-one

1-(4-acetoxy-3-(2-oxirany-methyl)phenyl)-pyrrolidin-2-one

1-[3-(3-chloro-2-hydroxypropyl)-4-hydroxyphenyl]-pyrrolidin-2-one

1-(1,3-benzodioxol-5-yl)-4-hydroxymethyl-pyrrolidin-2-one

22. A formulation as claimed in claim 1 for use in the treatment or prophylaxis of pain, inflammation or pyresis in a mammal.

23. A formulation as claimed in claim 1 characterised in that the compound of formula (I) is present in the range of from 1 to 100 mg/kg body weight of animal.

24. A compound of formula (IA), as claimed in either of claims claim 8, 9 or 10 for use in the treatment or proplylaxis of pain inflammation or pyresis in a mammal.

European Patent Office

# EUROPEAN SEARCH REPORT

0106988

Application number

EP 83 10 8640

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| P,X | EP-A-0 065 082 (THE WELLCOME FOUNDATION LTD.)<br>* Whole document *<br>--- | 1-3 | C 07 D 405/04<br>C 07 D 317/66<br>C 07 D 401/04<br>C 07 D 207/27<br>A 61 K 31/40 |
| A | EP-A-0 003 602 (F. HOFFMANN LA ROCHE)<br>* Claims; pages 1,2 *<br>--- | 1 | |
| A | US-A-4 297 440 (AOKI et al.)<br>* Column 12, lines 20-52 *<br>----- | 11-16 | |
| | | | **TECHNICAL FIELDS SEARCHED (Int. Cl. 3)** |
| | | | C 07 D 405/00<br>C 07 D 207/00<br>C 07 D 401/00<br>C 07 D 403/00 |

The present search report has been drawn up for all claims

| Place of search | Date of completion of the search | Examiner |
|---|---|---|
| THE HAGUE | 18-01-1984 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document